(19) Europäisches Patentamt
European Patent Office
Office européen des brevets

(11) Publication number: 0 048 455
A2

(12) # EUROPEAN PATENT APPLICATION

(21) Application number: 81107367.5

(22) Date of filing: 17.09.81

(51) Int. Cl.$^3$: C 12 N 15/00
C 12 P 21/02, C 12 N 1/20
C 07 H 21/04, A 61 K 39/135

(30) Priority: 18.09.80 GB 8030208
22.10.80 GB 8034130
27.11.80 GB 8038147
08.04.81 GB 8111064
18.08.81 GB 8125150

(43) Date of publication of application:
31.03.82 Bulletin 82/13

(84) Designated Contracting States:
BE CH DE FR GB LI NL SE

(71) Applicant: THE WELLCOME FOUNDATION LIMITED
183-193 Euston Road
London NW1 2BP(GB)

(71) Applicant: NATIONAL RESEARCH DEVELOPMENT CORPORATION
P.O. Box 236 Kingsgate House 66-74 Victoria Street
London SW1E 6SL(GB)

(72) Inventor: Boothroyd, John Charles
23 Clevedon Road
Penge London SE20 7QQ(GB)

(72) Inventor: Cross, George Alan Martin
37a The Grove
Biggin Hill Westerham Kent(GB)

(72) Inventor: Highfield, Peter Edmund
107 South Eden Park Road
Beckenham Kent(GB)

(72) Inventor: Winther, Michael David
Flat 3a 107, South Eden Park Road
Beckenham Kent(GB)

(72) Inventor: Rowlands, David John
Tile House 98 Busbridge Lane
Godalming Surrey(GB)

(72) Inventor: Brown, Fred
"Syndal" Glaziers Lane
Normandy Surrey(GB)

(72) Inventor: Harris, Timothy John Roy
70 Minley Road
Cove, Farnborough, Hants(GB)

(72) Inventor: Lowe, Peter Anthony
8 Gole Road
Pirbright, Surrey(GB)

(74) Representative: Berg, Wilhelm, Dr. et al,
Dr. Berg, Dipl.-Ing. Stapf, Dipl.-Ing. Schwabe, Dr. Dr.
Sandmair Mauerkircherstrasse 45
D-8000 München 80(DE)

(54) **Synthetic DNA and process therefor.**

(57) A DNA molecule comprising a nucleotide sequence substantially corresponding to all or a portion of foot and mouth disease virus RNA and in particular coding for at least one protein of foot and mouth disease virus. The DNA molecule can be inserted into a DNA cloning vehicle capable of expressing the DNA molecule, after a suitable host cell has been transformed by the cloning vehicle. The expression product can be incorporated into a vaccine for stimulating antibodies against FMDV. Methods for producing the DNA molecule, recombinant cloning vehicle and transformed host cell are described.

EP 0 048 455 A2

This invention relates to DNA molecules comprising artificially constructed polynucleotide sequences substantially corresponding to all or a portion of foot and mouth disease RNA, in particular it relates to polynucleotide sequences coding for at least one protein. It especially relates to DNA molecules comprising artificially constructed polynucleotide sequences coding for the whole or part of one or more proteins occurring in foot and mouth disease virus ( hereinafter referred to as FMDV ), or its precursor or a modification thereof. Such DNA molecules are capable of being expressed as a polypeptide(s).

Foot and mouth disease ( FMD ) is one of the most virulent and contagious diseases of farm animals. The disease is endemic in several areas of the world and can be found in many countries of Africa, Asia and South America where it is controlled to varying degrees by immunisation programmes. Countries which are free of the disease remain so only by strict import and quarantine controls, together with the use of slaughter when outbreaks occur.

FMDV is an RNA ( ribonucleic acid ) virus classified as a member of the genus <u>Apthovirus</u> of the family <u>Picornaviridae</u> ( see Cooper, P.D., et al, <u>Intervirology</u>, <u>10</u>, 165-180, 1978 ). There are seven known serotypes of FMDV, the European serotypes A, O and C, the South Africa Territories serotypes SAT 1, SAT 2, and SAT 3, and the Asia 1 serotype. A number of antigenically distinct subtypes are recognised within each of these serotypes, and as the subtypes are so distinct immunologically specific subtype vaccines are required. For each serotype or subtype several genetically distint variants exist.

Foot and mouth disease virus comprises a single strand of RNA and four major polypeptides, namely $VP_1$, $VP_2$, $VP_3$ and $VP_4$, which form the capsid proteins of the virus. The protein referred to here as $VP_1$ is variously referred

to by other workers in the field as $VP_3$, $VP_{Thr}$, and $VP_T$ ( see Bachrach, H.L. et al, J.Immunology, 115, 1636-1641, 1975; Strohmaier, K. et al, Biochem. Biophys. Res. Comm., 85, 1640-1645, 1978; Bachrach, H.L. et al, Intervirology, 12, 65-72, 1979 ). Each of the polypeptides $VP_1$, $VP_2$, and $VP_3$ has a molecular weight of about 26,000, whilst $VP_4$ has a molecular weight of about 8,000 and it is generally considered that there are approximately 60 copies of each of them in the virus. The other polypeptides translated from the virus RNA probably have a role in virus replication.

The single strand of FMDV RNA has a molecular weight of about 2.6 $\times 10^6$ which is equivalent to about 8000 nucleotides, and is of positive polarity acting as a template for both translation into polypeptides and RNA synthesis. One of the primary translational products is a protein designated P88 which is subsequently cleaved to produce the four capsid proteins $VP_1$ to $VP_4$.

Capsid protein $VP_1$, mentioned above, is susceptible to cleavage when intact virus is treated with trypsin, resulting in a large decrease in infectivity of most strains of FMDV ( Wild, T.F. and Brown, F., J. Gen. Virology, 1, 247-250, 1967 ). Trypsin treatment may also reduce the capacity of virus to stimulate the production of neutralising antibody. Thus it appears that $VP_1$, is likely to be the primary immunogen capable of eliciting effective protection against infection by FMDV and indeed $VP_1$ separated from virus particles produces neutralising antibody and elicits effective protection against the virus ( Laporte, J. et al, C. R. Acad. Sc. Paris, t. 276 Serie D, 3399-3401, 1973; Bachrach, H.L. et al, J. Immunology, 115, 1636-1641, 1975. ). Separation of the naturally occurring FMDV capsid proteins, particularly $VP_1$, in order to provide a safer vaccine has necessitated the use of strongly denaturing conditions and is generally held by those skilled in the art to be disadvantageous for the maintenance of optimum immunogenicity and production of an effective vaccine.

Using the techniques developed over the last five years it is now possible to introduce the DNA ( deoxyribonucleic acid ) coding for non-bacterial proteins into bacterial cells via the intermediary of a plasmid or other cloning vehicle, ( see for example Burrell, C. J, et al, Nature, 279, 43-47, 1979. ). In general the construction of the recombinant DNA molecules comprises the steps of deriving the DNA template coding for the desired protein from the non-bacterial parent and inserting this piece of heterologous DNA into a cloning vehicle, such as a bacterial plasmid, and then transforming an appropriate bacterial host with the modified plasmid. A general discussion of the manipulation of genes leading to the formation of recombinant DNA was published by S. Cohen in Scientific American, 233, 24-33, 1975.

Several non-bacterial genes have been inserted and multiplied within bacteria such as Escherichia coli, and several non-bacterial proteins have been expressed by bacteria using recombinant DNA technology, including the haemagglutinin of influenza viruses ( Porter, A. G. et al, Nature, 282, 471-477, 1979 ) and the hepatitis B virus protein ( Burrell, C. J et al, 1979, loc. cit.). Notwithstanding the considerable amount of work carried out in recent years on recombinant DNA research, there has been a considerable dearth of results amenable to immediate and practical application, especially in the field of recombinant DNA involving the manipulation of viral genetic material.

The present invention represents the first disclosure of the synthesis of individual polypeptides substantially equivalent to naturally occurring picornavirus capsid proteins, in particular FMDV capsid proteins, and also for the serial synthesis of several FMDV polypeptides either as individual entities or as fusion products of parts of two or more proteins. In addition, the present invention provides the means for the concurrent synthesis of immunogenic proteins normally encoded for by different variants of FMDV, without the numerous hazards associated with the culture of FMDV.

According to one aspect of the present invention there is provided a DNA molecule comprising a nucleotide sequence substantially corresponding to all or a portion of FMDV RNA or biologically functional fragments thereof, as hereinafter defined. Preferably the nucleotide sequence codes for at least one polypeptide of foot and mouth disease virus. Such a DNA molecule is capable of being expressed as a polypeptide recognisable as substantially corresponding to a FMDV protein. Preferably the nucleotide sequence codes for a structural protein of FMDV such as $VP_1$ or alternatively it may code for all of the structural or capsid proteins contiguously enabling them to be synthesised as one precursor, for example as P88 which may require stabilisation by inhibitors of the enzymes such as proteases which break it down to its constituent proteins. Alternatively the nucleotide sequence may code for $VP_1$ contiguous with the whole or part of any one of proteins $VP_2$, $VP_3$ and $VP_4$, in particular the sequence codes for $VP_1$ alone or together with the whole or part of $VP_3$. In yet another alternative the nucleotide sequence may code for all or a portion of at least two FMDV proteins each one derived from a different variant of FMDV.

In a preferred aspect the nucleotide sequence codes for a protein of FMDV serotype A or O, most preferably serotype A10 and in particular strain 61. Furthermore the nucleotide sequence may have control sequences positioned adjacent to it, such control sequences being derived either from FMDV nucleic acid or from a heterologous source.

In another aspect of the present invention there is provided a recombinant DNA molecule comprising an operon having initiator sequences and terminator sequences as hereinafter defined, and a nucleotide sequence substantially coding for all or part of at least one protein of foot and mouth disease virus, the nucleotide sequence being located between the initiator sequences and terminator sequences of the operon.

The invention also provides a recombinant DNA cloning vehicle capable of expressing all or part of a protein of FMDV comprising an operon having initiator sequences and terminator sequences, and a nucleotide sequence substantially coding for all or part of at least one portion of foot and mouth disease virus, the nucleotide sequence being located between the initiator sequences and terminator sequences of the operon.

In a further aspect of the invention there is provided a host cell containing a recombinant DNA cloning vehicle and/or a recombinant DNA molecule as defined above.

The invention also comprises an antigen for stimulating the production of antibodies against FMDV in a mammal comprising at least one polypeptide displaying FMDV immunogenicity prepared by the expression of a DNA molecule as hereinbefore defined and produced by a host cell transformed with a recombinat DNA cloning vehicle as hereinbefore defined and produced by a host cell transformed with a recombinant DNA cloning vehicle as hereinbefore defined.

The invention further provides a method of preparing a DNA molecule substantially coding for at least one polypeptide of FMDV comprising:

(a) isolating FMDV single stranded RNA;

(b) preparing a first single strand of DNA complementary to the single strand of FMDV RNA;

(c) preparing a second single strand of DNA complementary to and hydrogen bonded to the first DNA strand so as to produce a double strand of DNA.

The double strand of DNA so produced can be inserted into a cloning vehicle following digestion of the cloning vehicle with a restriction endonuclease, free ends of the cloning vehicle being joined to the DNA molecule so as to form a recombinant cloning vehicle. This in turn can be inserted into a suitable host cell by for example transformation.

As used herein the terms listed below have the following meanings:-

Nucleotide: a unit of DNA or RNA comprising a sugar moiety ( pentose ), a phosphate and a nitrogenous heterocyclic base. The base is joined to the sugar moiety via the glycosidic carbon ( 1' carbon of the pentose ) and the base characterizes the nucleotide. The four DNA bases are adenine ( A ), guanine ( G ), cytosine ( C ) and thymine ( T ). The four RNA bases are A, G, C and uracil ( U ).

Recombinant DNA :- a hybrid double stranded DNA sequence comprising at least two double stranded DNA nucleotide sequences, the first sequence not being found together in nature with the second sequence.

Cloning Vehicle :- non-chromosomal double stranded DNA capable of replicating when placed within a unicellular micro-organism.

Plasmid :- a cloning vehicle derived from viruses or bacteria.

Structural Gene :- a sequence of DNA nucleotides which codes for a sequence of amino acids characteristic of a specific polypeptide.

Initiator Sequences :- sequences of DNA nucleotides which control the initiation of transcription or translation.

Terminator Sequences :- sequences of DNA nucleotides at which transcription or translation ceases.

Transcription :- the process whereby RNA polymerase is caused to move along the DNA sequence forming messenger RNA.

Translation :- the process of producing a polypeptide from messenger RNA.

Operon :- a structural gene(s) coding for polypeptide expression which is preceded by initiator sequences and succeeded by terminator sequences.

Expression :- the process involved in producing a polypeptide from a structural gene.

Biologically functional fragment :- a DNA molecule which codes for antigenic determinants capable of eliciting an immune response in a mammal, or codes for a protein or part of a protein which is required for the appropriate confirmation of an antigenic determinant, or codes for a protein or part thereof which is important in the life cycle of FMDV in vivo and/or in vitro.

The invention will be further described by way of reference to the accompanying drawings in which:

Figure 1 is a schematic diagram of the genetic map of FMDV RNA and the proteins derived therefrom.

Figure 2 is a schematic diagram of the physical maps of three recombinant plasmids compared with the genetic map of FMDV RNA.

Figure 3 is a schematic diagram of the structural gene region of FMDV RNA and shows the FMDV portion of recombinant plasmid pFA61/t76 aligned with the structural gene region of FMDV.

Figures 4 to 7 are diagrams of the DNA and amino acid sequences in dicated by thick lines in Figure 3.

Figure 8 is a diagram comparing the $VP_1$ N-terminal amino acid sequences from five strains of FMDV.

Figure 9 is a diagram of C-terminal nucleotide sequence of $VP_3$ and the N-terminal nucleotide sequence of $VP_1$ in recombinant plasmid pFA61/t76.

Figure 10 is a diagram of the nucleotide and amino acid sequences at the junction of the region coding for $VP_2$ and $VP_3$ in recombinant plasmid pFA61/t76 which follows directly from the sequencing in Figure 5.

Figure 11 is a schematic diagram of the structural gene region of FMDV RNA and shows the FMDV portion of recombinant plasmid $pFO_1BFS/t251$ aligned with the structural gene region of FMDV.

Figure 12 is a diagram of the DNA sequence and predicted amino acid sequence of the FMDV structural gene region inserted into recombinant plasmid pFA A61/t76.

Figure 13 is a diagram of the DNA sequence and predicted amino acid sequence of $VP_4$; $VP_2$; $VP_3$ and $VP_1$ inserted into recombinant plasmid pFA A61/t 76.

Figure 14 is a schematic diagram of the construction of expression plasmid pXY1.

Figure 15 is a schematic diagram of the introduction of FMDV ds cDNA into plasmid pXY1.

Figure 16 illustrates the construction of expression plasmids.

Figure 17 illustrates the protein products and molecular weights of the proteins produced by the expression of the plasmids shown in Figure 16.

Figure 18 illustrates the C-terminal nucleotide and amino acid sequences of the proteins shown in Figure 17.

Figure 19 is a diagram of the nucleotide sequence of PFA61/t243 corresponding to the 3' end of FMDV RNA including at least part of the poly A tail.

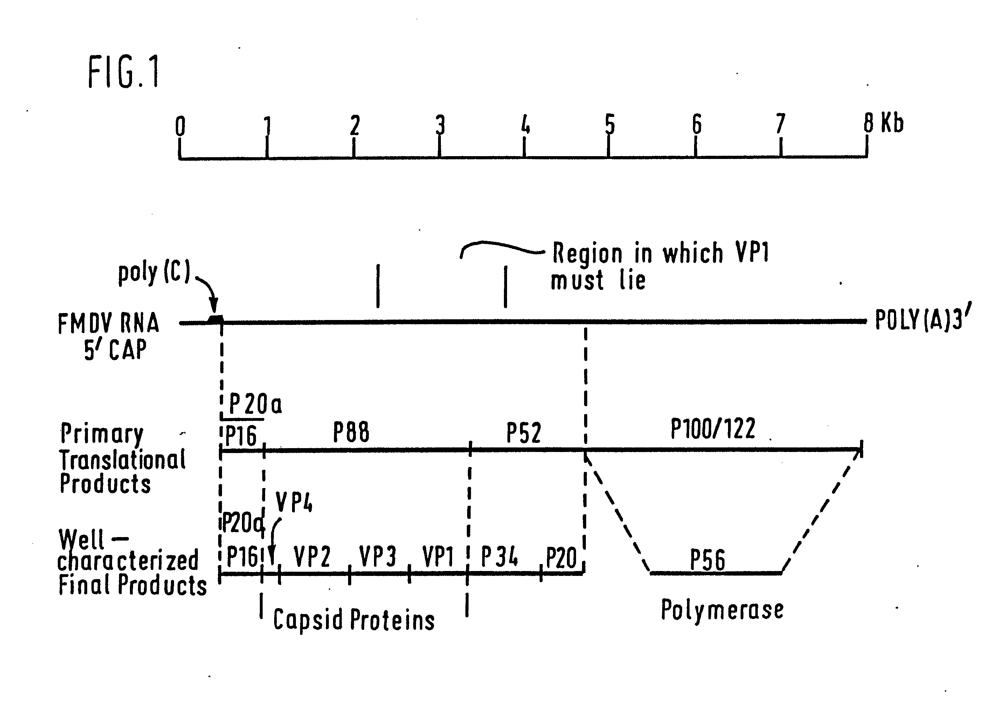

Referring to Figure 1, this shows a simplified map of FMDV RNA, which, as stated previously, is about 8000 nucleotides long. Translation of the RNA is from the 5' to 3' end. As with many messenger RNA molecules, FMDV RNA contains a polyadenylic acid sequence at the 3' end. It contains in addition a polycytidylic acid tract of about 100-200 nucleotides in length, depending on the strain of virus. The polycytidylic acid tract is located about 400 nucleotides from the 5' end of the RNA ( Rowlands, D J et al, J. Virology, 26, 335-343, 1978 ).

FMDV RNA is translated as a polyprotein, which is cleaved during translation to give several large polypeptides referred to as primary products, designated by "P" and a number. These primary products are then cleaved, probably by virus coded proteases into ultimate polypeptide products such as $VP_1$. There are portions of FMDV RNA which do not code for a protein but which play an important part in the life cycle of FMDV in vitro and/or in vivo, for example they affect the viability of the virus.

From the study of kinetics of translation of viral information into polypeptides in both virus infected cells and cell-free protein synthesising systems, the order on the viral genome of the genetic information encoding the major polypeptides has been deduced. ( Doel, T. R. et al, J. Gen. Viral., 41, 395-404, 1978; Sanger, D. V. et al, J. Virol, 33, 59-68, 1980 ). From these studies it has been found that the first 550 nucleotides of FMDV RNA, from the 5' end, do not appear to code for any polypeptide. Proteins P16 and P20a are closely related and contain the initiation site of FMDV translation as shown by exclusive labelling with N-formyl methionine during in vitro analysis ( Sanger, D.V. et al. 1980 loc. cit. ). P88 is the primary product which is split up into $VP_{1-4}$. From an evaluation of the prior art the gene for $VP_1$ is approximately 700 nucleotides and must lie within the region bounded by nucleotide positions 2300 and 3800.

A variety of techniques are available for preparing the recombinant DNA molecule according to the invention, one of which comprises the steps of synthesising a single stranded DNA copy (cDNA) of the RNA purified from an isolate of whole, virulent FMDV, using a reverse transcriptase enzyme. After the original RNA strand has been degraded the cDNA is converted into a double strand (ds cDNA), which is then treated to remove any loops of DNA which have formed using, for example, a nuclease enzyme. An alternative method of preparing the double stranded cDNA is via chemical synthesis using techniques well known in the art.

Once the double stranded cDNA has been produced the next step is to insert it into a cloning vehicle, which may be for example a bacterial plasmid or bacteriophage. This may be achieved by first cleaving the DNA of the purified cloning vehicle using a restriction endonuclease enzyme such as Pst 1, which cleaves the DNA at sites where complementary nucleotides are arranged in rotational symmetry. The double stranded FMDV ds cDNA can then be inserted between and linked to the open ends of the cloning vehicle by one of several methods. For example several "G" nucleotides can be attached to the 3' end of the cloning vehicle by a process called homopolymer tailing, which involves the use of a terminal transferase enzyme. In a similar fashion several "C" nucleotides are added to the 3' end of the double stranded FMDV cDNA. The tailed cloning vehicle and double stranded cDNA are then mixed together where upon the cohesive termini formed by the tailing anneal together.

There are several alternatives to 'tailing'. One involves digestion of ds cDNA with endonucleases forming either blunt or cohesive termini. Blunt termini may be made cohesive by exonuclease digestion. In another method, blunt ends may be directly joined using a DNA ligase enzyme. In yet another method, synthetic oligonucleotides may be joined to blunt ended ds cDNA and the new termini made cohesive by either exonuclease or endonuclease digestion, prior to ligation with appropriately linearized cloning vehicle.

Once the double stranded FMDV cDNA has been annealed with the DNA of the cloning vehicle, an appropriate host, such as a bacterium, is transformed with the recombinant cloning vehicle, so as to permit that host to express the FMDV ds cDNA, and thereby produce a polypeptide or polypeptides displaying FMDV antigenicity.

There are several host-cloning vehicle combinations that could be used for the expression of FMDV proteins. For example useful cloning vehicles include bacterial plasmids such as pAT 153, ( Twigg, A.J. Nature, 283, 216-218, 1980; obtainable from Prof. D. Sherratt, University of Glasgow, Scotland. ), pBR 322, ( Sutcliffe, J. G. Cold Spring Harbour Symposium for Quantitative Biology, 43, 77-90, 1978; obtainable from Dr. H. Boyer, University of San Francisco, U.S.A. ), other E. coli plasmids and wider host range plasmids. Bacteriophages such as the many derivatives of phage $\lambda$ may also be suitable. Hosts that may be used include bacteria such as strains of E. coli K. 12, e.g. E. coli HB101, ( Boyer, H. W. et al, J. Mol. Biol., 41, 459-472, 1969; obtainable from Dr. H. Boyer, University of San Francisco, U.S.A. ), E. coli $\chi$1776, ( Curtiss, R. et al, Ann Report Dept. of Microbiology University of Alabama, 1976, 96-106; obtainable from Dr. R. Curtiss III, University of Alabama, Birmingham, USA ), E. coli $\chi$2282 ( obtainable from Dr. R. Curtiss III ) and E. coli MRC1, ( obtainable from Dr. S. Bremer, University of Cambridge, England ), strains of Bacillus subtilis and Pseudomonas, as well as yeasts and other fungi, and other unicellular organisms. It is only to be expected, however, that not all hosts will be equally effective

Within each cloning vehicle, various sites may be available for insertion of the FMDV ds cDNA, each site being designated by the restriction endonuclease enzyme which cleaves the DNA. Thus, for example, enzyme Pst 1 cleaves plasmid pAT 153 in the gene coding for penicillin resistance. There are several other endonuclease enzymes that can be employed including Hind III and Eco RI.

The selection of the site on the cloning vehicle for insertion of FMDV ds cDNA may be governed by a variety of factors, for example size of polypeptide to be expressed and location of initator and terminator sequences. Consequently not all sites may be equally effective for a given polypeptide. Selection of the site may also be governed by the initial screening method employed for detecting recombinants, for example as stated Pst 1 cleaves plasmid pAT 153 in the gene coding for ampicillin resistance, thus colonies of transformants displaying tetracycline resistance but ampicillin sensitivity are likely to contain at least some FMDV ds cDNA.

It is essential that the FMDV ds cDNA inserted into the cloning vehicle can be read in the correct phase. In order to achieve this it may be necessary to insert supplementary nucleotides for example between the starting points of transcription and translation of the FMDV ds cDNA fragment whose expression is desired. Addition of such nucleotides must not, of course, form a nucleotide sequence that could interrupt translation.

Expression of FMDV ds cDNA, which has been inserted into a cloning vehicle, which in turn has been used to transform a suitable host cell, may be detected by the appearance of a function specific for the protein, that is immunological activity in the case of FMDV. Several methods are available, for example the essentially immunological colony screening method disclosed by S. Broome and W. Gilbert in Proc. Nat. Acad. Sci., 1978, 75, 2746-2749. One alternative, and somewhat simpler technique, is to inject into a laboratory animal the crude bacterial extract derived from a culture of bacteria transformed with an appropriately engineered cloning vehicle and to test for the formation of appropriate antibodies. A second alternative is to perform an immunoprecipitation of a crude extract of the bacterial cells. Yet a further method is the "Maxicell Technique" described by Sancar et al in J. Bact., 1979, 137, 692-693.

The nature of the polypeptide produced as a result of expression by the host of the recombinant DNA molecule of the invention will depend on the point of insertion into the DNA of the cloning vehicle, so that in practice a precursor polypeptide may be formed which comprises a polypeptide coded for by FMDV ds cDNA and an additional polypeptide coded for by the DNA of the cloning vehicle. Thus, for example, if the plasmid pAT 153 is cleaved by Pst 1 a precursor polypeptide comprising a portion of the penicillinase enzyme and the polypeptide coded for by the FMDV ds cDNA may be expressed. The precursor polypeptide may then be selectively cleaved so as to separate the desired FMDV polypeptide from the superfluous amino acid sequence. Usually cleavage will be effected outside the host following harvest of the microbial culture by techniques well known to those skilled in the art. Cleavage may be necessary in order that the FMDV expression product can exert the desired activity, however during harvest of the microbial culture the fact that a superfluous amino acid sequence is linked to the required FMDV polypeptide may help to prevent degradation of the expression product by endogenous enzymes. Alternatively cleavage may be effected within the host, this may be achieved by inserting into the cloning vehicle, DNA coding for the desired cleavage enzymes.

The production of a fusion product of a polypeptide coded for by FMDV ds cDNA and a portion of for example penicillinase enzyme may increase the stability of the hybrid protein in E. coli and even enhance the immunogenicity of the FMDV protein.

Alternatively, appropriate nucleotide sequences, derived for example from FMDV RNA, may be inserted before the FMDV ds cDNA so as to ensure that the FMDV ds cDNA can be expressed alone, and not as a fusion product with a host polypeptide.

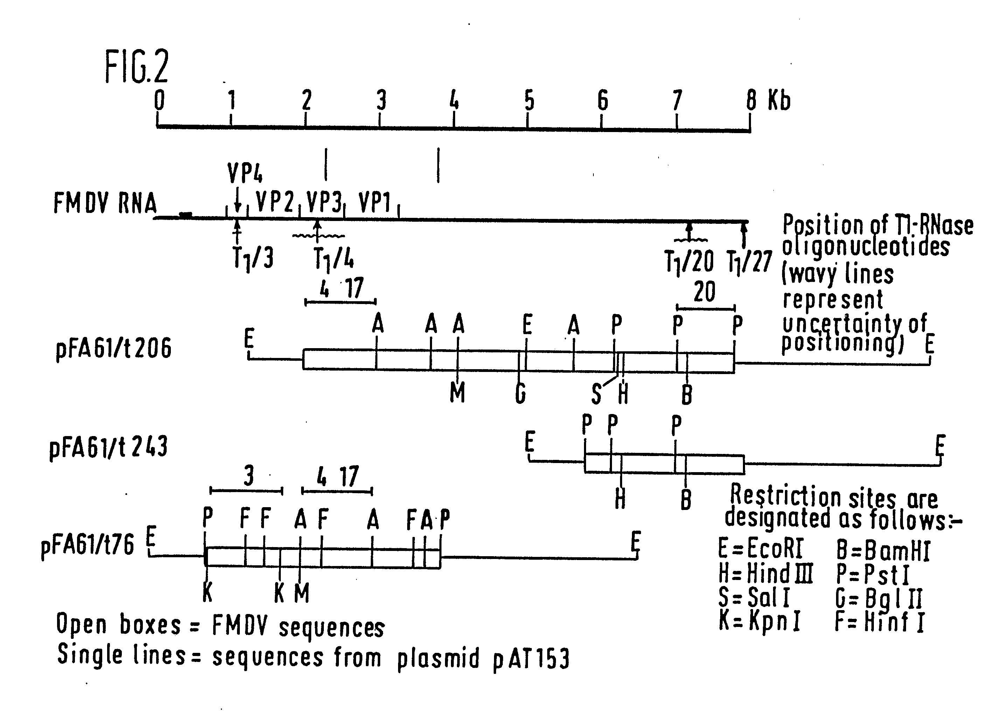

Once transformants containing at least some of FMDV ds cDNA have been identified, as explained above, the recombinant cloning vehicle DNA is purified and then analysed in order to determine how much of the FMDV ds cDNA has been inserted. In order to do this the recombinant cloning vehicle DNA is treated with several different restriction endonucleases, for example Eco RI, Pst 1, Sal 1, Bgl II, Bam H1, Hind III and Hinf 1, and the digestion products may be analysed by gel electrophoresis. Using these results, together with those obtained when T1 oligonucleotides, produced by RNase digestion of FMDV RNA by T1 nuclease, are allowed to hybridize with separated restriction endonuclease digestion products of the recombinant DNA, maps of the FMDV ds cDNA inserted into the cloning vehicle, similar to those shown in Figure 2, may be produced. From such maps it is possible to select the recombinant that contains the desired FMDV gene, for example the gene coding for $VP_1$.

In another aspect of the invention there is provided a method of mapping the recombinant DNA molecule as hereinbefore defined comprising:

(a) digesting the DNA with restriction endonuclease enzymes and separating the products;

(b) adding labelled olignucleotides of FMDV RNA produced by $T_1$ ribonuclease digestion;

(c) identifying the products of restriction endonuclease digestion to which the $T_1$ oligonucleotides have hybridlized.

The various DNA molecules may be useful as a probe for the in vitro diagnosis of the presence in biological samples of FMDV, and in particular may be used to determine the serotype or subtype causing an outbreak of FMD. For this purpose the DNA molecules may be labelled, in known manner, with a radioactive isotope. In addition the expression product of the recombinant cloning vehicle may be used for serological diagnosis and in the preparation of single or multivalent vaccines against FMD, by methods well known in the art of vaccine manufacture. Such vaccines are effective in stimulating antibodies in vaccinated animals and thereby protecting against FMD.

In another aspect of the invention there is provided a vaccine for stimulating the production of antibodies against FMDV in a mammal comprising at least one protein displaying FMDV immunogenicity produced by a host cell as hereinbefore defined together with a veterinarily acceptable carrier therefor.

In yet a further aspect of the invention there is provided a method of stimulating the production of antibodies against FMDV in a mammal, comprising administering an immunologically effective non-toxic amount of a vaccine as defined above. The term "immunologically effective non-toxic amount" is used to denote an amount of protein displaying FMDV immunogenicity sufficient to stimulate enough antibodies in a mammal, such that if the mammal encounters virulent FMDV, following vaccination, it does not succomb tó the disease, and which is not toxic to the mammal.

Further characteristics and features of the invention are described in the following Examples which are presented by way of illustration only and are not to be considered as limiting the scope of the present invention in any way.

EXAMPLE 1

Preparation of FMDV RNA

Approximately 10 ml of a recent harvest of FMDV type A10 ( strain A61) (freely available on application to Animal Virus Research Institute, Pirbright, England, subject to the requirements of the law of individual countries) in Eagle's medium were added to each of ten Roux bottles containing monolayers of approximately $10^8$ $BHK_{21}$ cells. After gentle shaking for 30 min the medium was decanted and 20 ml fresh medium added to each bottle. After the virus infection had destroyed the cell monolayers ( 3-4h ), the medium was decanted and the cell debris was removed by centrifugation at 12,000xg for 15 min at 4°C. The virus was then pelleted by spinning at 90,000xg for 1hr at 4°C. The virus pellet was resuspended in 2 ml of TNE buffer ( 10mM Tris-HCl ( pH 7.5) 150mM NaCl and 1mM EDTA ( ethylenediamino tetracetic acid ) and the suspension was cleared by centrifuging for 10 min at 20°C at about 5,000xg. The cleared supernatant was made 1% w/v in sodium dodecyl sulphate ( SDS ) and loaded onto a preformed gradient ( sucrose 15-45% w/v ) in TN buffer ( 100mM Tris-HCl pH 7.6, 100mM NaCl ) and spun at 100,000xg for 2hr at 10°C. Fractions ( 1 ml ) were monitored at 260nm and the fractions containing virus were extracted once with an equal volume of phenol:chloroform ( 1:1 ) and the aqueous phase precipitated by the addition of 2 volumes ethanol, incubating overnight at -20°C. The RNA precipitate was pelleted by spinning at 5,000xg for 30min at 4°C. The supernatant was discarded, the pellet drained and then dissolved in 0.5 ml TNES buffer ( 10mM Tris-HCl pH 7.6, 150mM NaCl, 1mM EDTA, and 0.2% w/v SDS ). This solution was loaded onto a preformed sucrose gradient ( 5-25% ) in TNES and centrifuged at 200,000xg for 3.5hr at 20°C. Fractions ( 0.5 ml )containing RNA sedimenting at 35S were pooled, phenol-chloroform extracted once, ethanol precipitated and redissolved as described for virus above. The resulting solution was ethanol precipitated and finally dissolved in 0.05 ml of double distilled water.

Synthesis of double stranded complementary DNA ( DScDNA )

FMDV RNA ( 10 µg ) and oligo-$dT_{(12-18)}$primer ( 0.5 µg; obtained from Collaborative Research ) were incubated at 30°C for 2 hr in a final volume of 100 µl containing 0.4mM dithiothreitol, 8mM $MgCl_2$, 50mM Tris-HCl pH 8.1 148mM NaCl, 0.2mM dATP ( 2.5Ci/m mole [$\alpha$-$^{32}$P]-dATP; obtained from Radiochemical Centre, Amersham ), 0.2mM dTTP, 0.2mM dCTP, 0.2mM dGTP, 4mM $Na_4P_2O_7$ and 28 units AMV reverse transcriptase ( supplied by Dr. J. Beard, Life Sciences Inc., Florida ). The reaction was stopped by adding EDTA ( 20mM ) and SDS ( 0.2% w/v ). A sample ( 2% ) of this terminated reaction mixture was spotted on 2.5cm DE81 paper discs ( obtained from Whatman ). These were washed extensively in 5% ( w/v ) $Na_2HPO_4$, followed by a brief wash ( 5 min ) in distilled water before drying and scintillation counting. From this procedure, a total yield of approximately 2 µg of cDNA was calculated. The remainder of the reaction mixture was phenol-chloroform extracted and ethanol precipitated as described above except that sodium acetate was added to 0.2M before addition of the ethanol. The desiccated pellet was dissolved in 100 µl 0.1M NaOH and incubated at 70°C for 20 min to remove the template i.e. FMDV RNA. The solution was neutralised with acetic acid and the cDNA resolved from degraded RNA by passing through a column ( 100 x 15mm ) of bead-f phenol-chloroform. The desiccated pellet was resuspended to a final volume of 90 µl in 50mM Tris-HCl pH 8.3 20mM dithiothreitol, 10mM $MgCl_2$,

0.4mM dCTP, 0.4mM dGTP, 0.4mM dATP ( 5.5Ci/m mole [$\alpha$$^{32}$P]-dATP; Radiochemical Centre, Amersham ), 0.4mM dTTP, and 36 units AMV reverse transcriptase. After 4 hr incubation at 45°C the reaction was stopped by the addition of EDTA ( 20mM ) and SDS ( 0.2% w/v ). The mixture was phenol-chloroform extracted, NaCl was added to 0.2M and the mixture precipitated with 2 volumes of ethanol overnight at -20°C. The desiccated pellet was resuspended in 200 µl of 50mM NaCl, 0.1% w/v SDS and passed through a Sephadex G100 column as described above. The excluded peak was ethanol precipitated in the presence of 200 µg glycogen carrier. DE81 paper disc analysis, as described above, indicated the second strand synthesis was about 60% of the maximum theoretical yield. The desiccated pellet from ethanol precipitation was resuspended in a final volume of 200 µl S1 buffer ( 25mM sodium acetate ( pH 4.6 ), 150mM NaCl, and 1mM $ZnSO_4$ ). A 28 µl sample of this ( sample A ) was stored at -20°C while the remainder ( sample B ) was incubated with 5 units $S_1$ nuclease ( obtained from Sigma ) for 30 min at 37°C. The reaction was stopped by extraction with phenol-chloroform and the aqueous phase precipitated with ethanol. The desiccated pellet was resuspended in 20 µl $H_2O$. To check the effectiveness of the S1 nuclease treatment in removing the loops in the double-stranded cDNA, a small amount ( 2% ) of samples A and B was heated in S1 buffer at 100°C for 6 min, cooled to 60°C and 3 units of S1 added at varying times ( 0, 0.25, 1 and 14 hr ) followed by incubation at 37°C for 30 min. Resistance to S1 was assayed by DE81 paper disc binding as described above. The results showed that greater than 53% of sample A was resistant to S1 digestion compared with less than 2% of sample B when both were incubated with S1 immediately upon cooling to 60°C. If S1 was added 0.25h after the samples were placed at 60°C these figures rose to 81% and 15%, respectively. Hence the initial S1 treatment had effectively cleaved the loops present after second strand synthesis.

An estimate of the size of the molecules in sample B by agarose gel electrophoresis showed them to be distributed between full length copies ( i.e., approximately 8000bp [base pairs] ) down to less than 200bp, with a mean value of 2,000-4,000bp.

Homopolymer tailing of plasmid and double stranded cDNA ( ds cDNA )

( a ) dG-tailing of the vector. Approximately 7 μg of the vector, plasmid pAT153, were digested with Pst 1 endonuclease under conditions recommended by the enzyme suppliers ( Boehringer ). The reaction was stopped by the addition of sodium acetate to 0.3M and ethanol precipitated. The desiccated pellet was resuspended in 100 μl of dG-tailing buffer ( 100mM sodium cacodylate-HCl pH 7.1 ), 5mM $MgCl_2$, 50μg/ml bovine albumin and 1mM dGTP ( 200mCi/[8$^3$-H]-dGTP/m mole ). To this, 2 μl ( 38 units ) of terminal transferase ( TT) were added and the reaction incubated at 37$^o$C. Samples were removed after 2.5, 5 and 10 min. In each case the reaction was stopped by chilling on ice and adding EDTA to 20mM. Analysis by trichloroacetic acid precipitation of a 5 μl sample from each showed that no further incorporation of $^3$H dGMP occurred after 2.5 min and that the average length of the homopolymer tail by this time was about 25 nucleotides. The remainder of the 2.5 min sample was diluted to 100 μl with TE buffer ( 10mM Tris-HCl pH 8.0, 1mM EDTA) and extracted once with an equal volume of TE-saturated phenol. The aqueous phase was extracted four times with ether; sodium acetate was added to 0.3M and the sample ethanol precipitated. The desiccated pellet was resuspended in 40 ul $H_2O$ and stored at -10$^o$C. The concentration of DNA in this final solution was estimated to be 15μg/ml.

( b ) dC-tailing of dscDNA. Approximately 0.4μg of dscDNA in dC-tailing buffer ( 100mM sodium cacodylate HCl ( pH 7.1 ), 1mM $CoCl_2$, 0.1mM DTT, 50μg/ml bovine serum albumin 0.5mM $^3$H dCTP ( 600mCi/[5-$^3$H]-CTP/m mole )

was incubated with 1 μl TT ( 19 units ). Aliquots were removed after 2 and 5 min and assayed for incorporation by TCA precipitation of a 5 μl sample of each. This indicated the average length of the homopolymer tail to be about 70 nucleotides after 2 min and 160 nucleotides after 5 min. These two aliquots were pooled, phenol extracted once, ether extracted four times, ethanol precipitated, redissolved in 50 μl $H_2O$ and stored at -10°C.

Transformation with annealed plasmid and dscDNA

Approximately 0.12μg of dG-tailed plasmid pAT153 ( 0.05pmoles ) and 0.1μg dC-tailed dscDNA ( 0.2-0.4pmoles ) were incubated at 65°C for 0.5h in 100μl TNE Buffer ( 10mM Tris-Cl pH 8.0, 200mM NaCl, 1mM EDTA ). The temperature of the incubation was steadily dropped to 20°C over a 4 hour period and then rapidly brought to 0°C. The solution was diluted to 200μl containing, finally, 15mM Tris-HCl pH 7.5, 100mM NaCl, 10mM $MgCl_2$, 10mM $CaCl_2$, 0.5mM EDTA and stored at 0°C for about 3 hours.

A transformation competent culture of E.coli HB101 was prepared by inoculating 1ml of a stationary phase culture into 65ml L-broth ( 1% Difco Bacto Tryptone, 0.5% Difco Bacto Yeast Extract and 0.5% NaCl, pH 7.2 ) and shaking at 37°C for about 3h after which time the $A_{650}$ was 0.4. The cells were pelleted by spinning at 3,000xg for 5 min at 4°C. The cells were resuspended in 25ml 0.1M $MgCl_2$ ( 0°C ) and repelleted. The pellet was resuspended in 2ml 0.1M $CaCl_2$ ( 0°C ) and left for 30min at 0°C. Approximately 0.2ml of such transformation-competent cells were gently mixed with 0.1ml of annealed pAT153/dscDNA and left for a further 30min at 0°C, followed by 2min at 42°C, and a final 30min at 0°C. To this was added 1ml L-broth and the transformation mix then incubated at 37°C for 25min. Ten 0.1ml samples of this were spread onto L-Tc plates ( L-broth plus 1.5% agar and 15μg/ml tetracycline ( obtained from Sigma ) ) and incubated overnight at 37°C. From these ten plates 150 tetracycline resistant colonies were obtained of which about 87% were sensitive to ampicillin ( 100μg/ml; Sigma ) suggesting insertion of cDNA at the Pst 1 site of pAT153.

Large scale recombinant plasmid preparation

The transformants containing the plasmids were grown in bulk by inoculating 10ml stationary culture into 500ml L-broth and shaking at 37°C for about 4h until $A_{590}$ reached about 1.0. Chloramphenicol ( obtained from Sigma) was then added to 0.1mg/ml and the cultures shaken at 37°C overnight. After this amplification step the cells were pelleted by centrifuging at 5000xg for 5min at 4°C. The supernatant was disinfected and discarded, and the cell pellet resuspended in 12ml R buffer ( 50mM Tris-HCl ( pH 8,0 ), 40mM EDTA, 25% sucrose ). Lysosyme and EDTA were then added to 1.4mg/ml and 60mM respectively and the suspension left on ice for 5min. To 16ml of this were added 30ml Triton mix ( 0.1% Triton X-100, 62.5mM EDTA, 50mM Tris-HCl pH 8.0 ) and the mixture left on ice about 10min or until viscous. The mixture was then cleared by spinning at 48,000xg for 15min at 4°C. The resulting supernatant was carefully decanted and 0.95g CsCl and 0.1ml of a 10mg/ml solution of ethidium bromide added per ml. This was spun at 12000xg in a Beckman 50Ti rotor at 20°C for 40h. The plasmid band was visualised by long wave U.V. fluoresence and removed by syringe by piercing the side of the tube. The plasmid DNA was spun to equilibrium in a second CsCl/ethidium bromide gradient. The resulting band was extracted 4 times with propan-2-ol saturated with NaCl and $H_2O$, and then ethanol precipitated, redissolved in TE and reprecipitated. The final desiccated pellet was resuspended in 200μl TE buffer and stored at 4°C.

Physical mapping of recombinant plasmids using restriction endonucleases

The restriction endonucleases Eco RI, Pst I, Sal I, Bgl II, and Sma I were purchased from Boehringer; Bam HI, Hind III, Hinf I, Ava I, Xba I and Hinc

II were purchased from Bethesda Research Laboratories and Kpn I was purchased from New England Biolabs. The purified recombinant plasmid DNA was digested by these enzymes using reaction conditions specified by the suppliers. The digestion products were analysed by electrophoresis through either a 1% agarose or a 7% acrylamide gel and were visualised by ethidium bromide staining and U.V. fluorescence. Bacteriophage lambda DNA digested with Hind III ( Boehringer ) and plasmid pAT153 digested with Hinf I were used as size markers.

Orientation and mapping of FMDV DNA inserted into plasmids using TI ribonuclease oligonucleotides derived from FMDV

RNase T1 oligonucleotides, labelled with $^{32}P$ at the 5' end for use as probes, were prepared by two-dimensional gel electrophoresis. The oligonucleotides were either labelled before electrophoresis by the procedure of Harris ( T J R Harris; Nucleic Acids Research, 7, 1765-1785 1979 ) using 1-5μg of RNA and 1 unit of RNase T1, or were isolated from a two dimensional gel and then end-labelled. In the latter procedure, an RNase T1 digest of 100μg of RNA labelled *in vivo* with $^{32}P$ ( D J Rowlands, T J R Harris & F Brown 1978 *loc cit* ) was separated by two dimensional gel electrophoresis and an autoradiograph of the gel used to locate the oligonucleotides. In each procedure the oligonucleotides were eluted from the gel pieces by the crush and soak method ( A Maxam & W Gilbert: Proceedings of the National Academy of Sciences, USA, 74, 560-564 1977 ). The order of the oligonucleotides on the genome is described by T J R Harris, K J H Robson & F Brown ( J General Virology, 1980, in press).

Purified recombinant DNA was digested with restriction endonucleases, the products separated on a 1% agarose gel and then transferred to a nitrocellulose filter by the method of Southern ( E M Southern: J Molecular Biology, 98, 503-513 1975 ). The filter was preincubated in hybridization buffer (40% formamide, 0.4M NaCl, 10mM PIPES-NaOH pH 6.4, 100μg/ml *E.coli* tRNA, 0.5% SDS, 0.04% Ficoll [synthetic high molecular weight polymer of sucrose and epichlorohydrin], 0.04% polyvinylpyrrolidone 400, 0.04% bovine albumin ) at 50°C for 2h in a sealed plastic bag before addition of labelled RNase T1 oligonucleotides ( 75-150,000cpm ). After 16h, the filter was washed at 40°C in 2xSSC (8x250ml over 6h), air-dried and then autoradiographed at -70°C using Fuji XR film and intensifying screen.

Results

The physical maps of three recombinant plasmids, pFA61/t206, pFA61/t 243 and pFA61/76 and their alignment and correspondence with the genes of FMDV, as deduced by application of the above methods, are shown in Figure 2.

The orientation of the largest recombinant pFA61/t206, containing a sequence of about 5,800 nucleotide pairs corresponding to about 73% of the FMDV RNA sequence, was first determined using ribonuclease T1 oligonucleotides derived from and previously mapped on the virus RNA ( Harris et al, 1980 loc. cit. ). T1 oligonucleotides numbers 3, 4 and 20 and 27 were particularly useful for this purpose owing to the accuracy ( indicated on Figure 2 ) to which their position in FMDV RNA is known. The alignment of other recombinants ( for example, pFA61/76 ) relative to recombinant pFA61/t206 and to FMDV RNA was determined by mapping of restriction endonuclease fragments of the recombinants and hybridization with T1 oligoribonucleotides derived from the virus RNA. Two of the recombinants shown in Figure 2 clearly contain the genetic information for several FMDV proteins including $VP_1$.

Recombinant pFA61/t206 contains $VP_1$ in an orientation for the expression of a polypeptide consisting of a fusion product of the N-terminal part of plasmid-encoded β-lactamase and a FMDV determined polypeptide.

a) EXPRESSION

Construction of an expression vector, pXY1

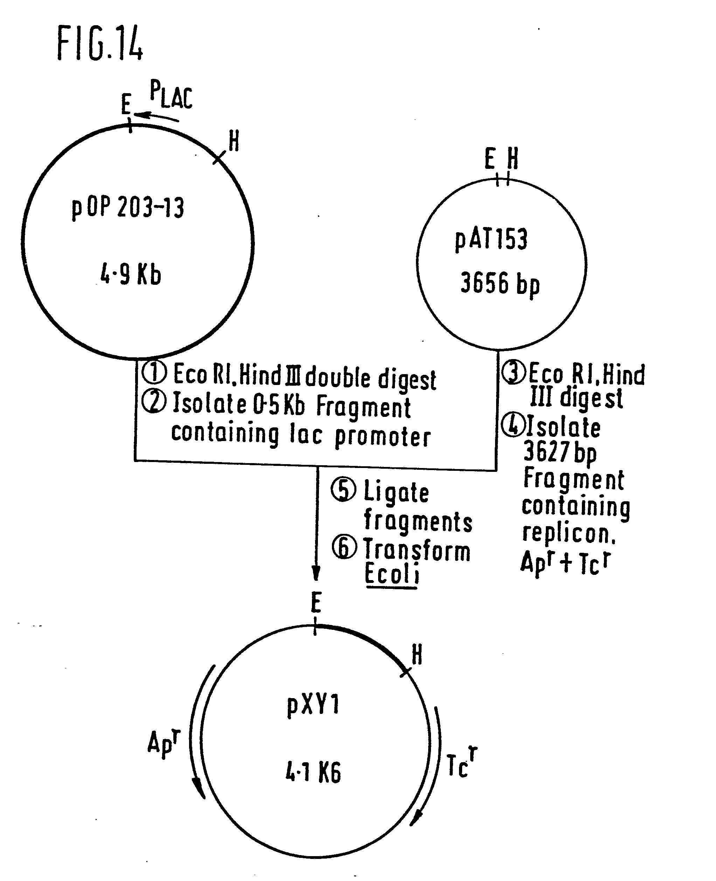

A large scale recombinant plasmid preparation of pOP 203-13 (obtainable from Forrest Fuller, Harvard University, U.S.A.) was carried out using the techniques described above. 1 µg of the purified plasmid DNA was digested with 5 units each of ECoRI and Hind III for 1 hour at 37°C. 1 µg of the plasmid pAT153 was also digested with 5 units each of EcoRI and Hind III as above. Both DNA samples were then run on an agarose gel. The 0.5 kb (kilobase pairs) DNA fragment of pOP 203-13 and the 3.6 Kb DNA fragment of pAT153 were cut out of the agarose gel and extracted by the method of Vogelstein and Gillespie, Proc. Nat. Acad. Sci., 76, 615-619, 1979. The purified DNA fragments were mixed and ligated with 0.1 unit of T4 DNA ligase (Miles) for 16 hours at 15°C. The ligated DNA was used to transform a competent culture of E. coli. HB101 (prepared as described above) and the cells were plated out on L-Ap plates (L-broth plus 1.5% agar and 100µg/ml ampicillin (obtained from Sigma) and incubated overnight at 37°C. Many recombinants were obtained, one of which was further characterised by restriction mapping and was designated pXY1 (figure 14). Fragments of FMDV ds cDNA cloned into the unique EcoR1 site of pXY1 can result in the expression of a hybrid protein consisting of the first eight amino acids of β-galactosidase, 2 amino acids from the EcoR1 site and a number of amino acids coded for by the introduced FMDV ds cDNA fragment. Hybrid proteins so produced will be under the control of the lac operon. Transcription can be induced (increased) by the addition of 30 µg/ml IPTG (isopropyl 1-thio-β-D-galactopyranoside) when the recombinant plasmid is in a host bacterium containing sufficient quantities of specific repressor protein. This can be achieved through introduction of an F' factor containing a Lac $I^q$ mutation into the host bacterium (see Henning et al, Proc. Nat. Acad. Sci., 76, 4360-4364, 1979).

b) Introduction of FMDV ds cDNA into pXY1

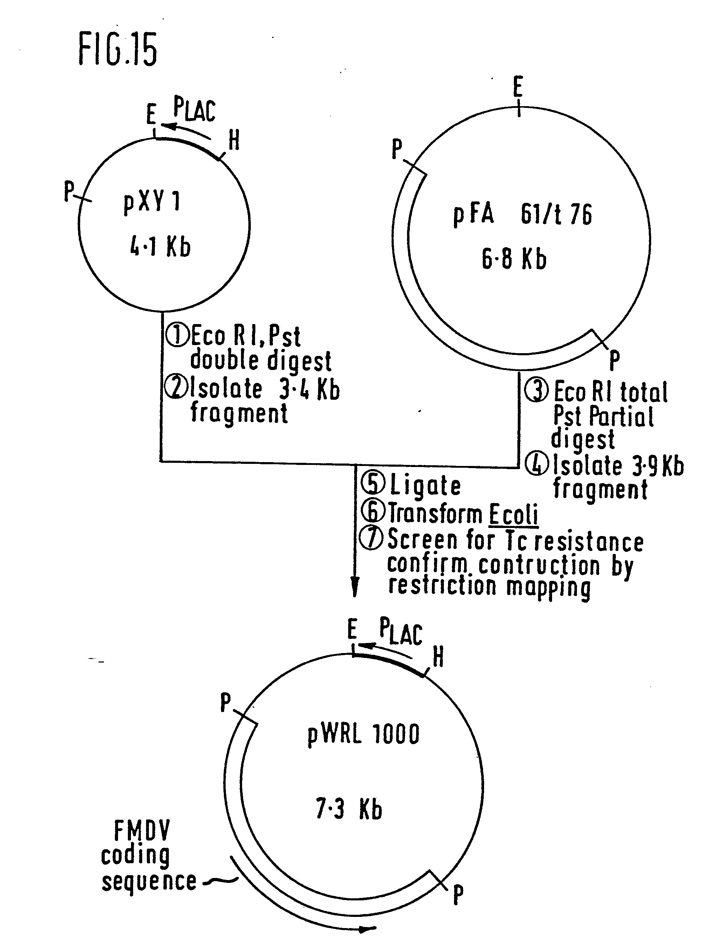

2 μg of pFA61/t76 prepared as described above was digested with 6 units of EcoR1 and 1 unit of Pst I for 1 hour at 37°C. This resulted in a complete digestion with EcoR1 but only a partial digestion with Pst I. The 4.0 Kb partial digestion product containing the entire cDNA insert was isolated on an agarose gel as described previously. This was added to 2.0 μg of pXY1 (cut with 2 units each of EcoR1 and Pst I) and the DNA fragments were joined together by incubating with T4 DNA ligase (0.05 unit) for 16 hours at 15°C. The DNA was used to transform competent cultures of E. coli HB101 which were subsequently grown on L-Tc plates (L-broth plus 1.5% agar and 10 μg/ml tetracycline). Many recombinants were obtained that were ampicillin-sensitive, indicating the insertion of a DNA fragment into the Pst site of pXY1. One such recombinant plasmid was further characterised by restriction mapping and designated pWRL 1000 (figure 15). This plasmid contained the lac promoter and the FMDV cDNA sequences coding for p88 in the correct orientation

c) Construction of expression plasmid, pWRL 1004

To achieve expression of FMDV sequences it was necessary to remove the DNA segments separating the FMDV cDNA from the lac promoter. 1 μg of purified pWRL 1000 was digested with 10 units of Sac II and ethanol precipitated. The dry DNA was redissolved in 0.1 ml of buffer (0.1 M NaCl, 0.005 M $MgCl_2$, 0.005 M $CaCl_2$, 0.02 M Tris-HCl, pH 8.1, 0.001 M EDTA) and reacted with 0.2 units of nuclease Bal 31 (BRL) at 18°C for 15 minutes. The reaction was stopped by the addition of 5 μl 0.5 M EDTA. Under these conditions Bal 31 removes an average of 300 bp DNA from the ends of the linear double-stranded DNA. After ethanol precipitation the DNA was digested with 5 units of EcoR1 and again ethanol precipitated. The DNA was taken up in 40 µl Polymerase I buffer (0.06 M tris-HCl, pH 7.5, 0.008 M $MgCl_2$, 0.2mM each of d ATP, dTTP, dCTP, dGTP, 0.01 M B-mercaptoethanol, 1mM ATP and 1.4 units E. coli DNA polymerase (large fragment) and incubated 10 minutes at 10°C. The blunt ends of the linear plasmid were then ligated together with 0.4 units of $T_4$ DNA ligase for 16 hours at 15°C and used to tranform E. coli AB2480 (obtained from P. Emmerson, University of Newcastle, U.K.) containing F' Lac $I^q$. A number of recombinants were obtained in L-Tc plates. One of the recombinants was characterised by restriction mapping and designated pWRL 1004. As a result of the restriction mapping pWRL 1004 was found to have regenerated an EcoR1 site at the junction of the lac promoter and the FMDV cDNA sequences. The plasmid-encoded proteins were examined in the UV-sensitive strain AB2480 by the "Maxicell Technique" (Sancar et al, J. Bact., 137, 692-693, 1979) and pWRL 1004 was found to synthesize large proteins ( 50,000 daltons) coded for by the FMDV cDNA.

d) Construction of additional expression plasmids

1 µg of pWRL 1004 was digested with 6 units of Pvu II and ligated with $T_4$ DNA ligase in the presence of 0.2 µg of synthetic DNA fragments (containing a sequence recognized by EcoR1). The introduction of the EcoR1 "linker" (obtained from Collaborative Research) results in the introduction of a "Stop" codon four amino acids away from the C-terminus of $VP_1$. This plasmid was designated pWRL 1120 and produced a 29,000 dalton protein containing $VP_1$ when examined in "Maxicells".

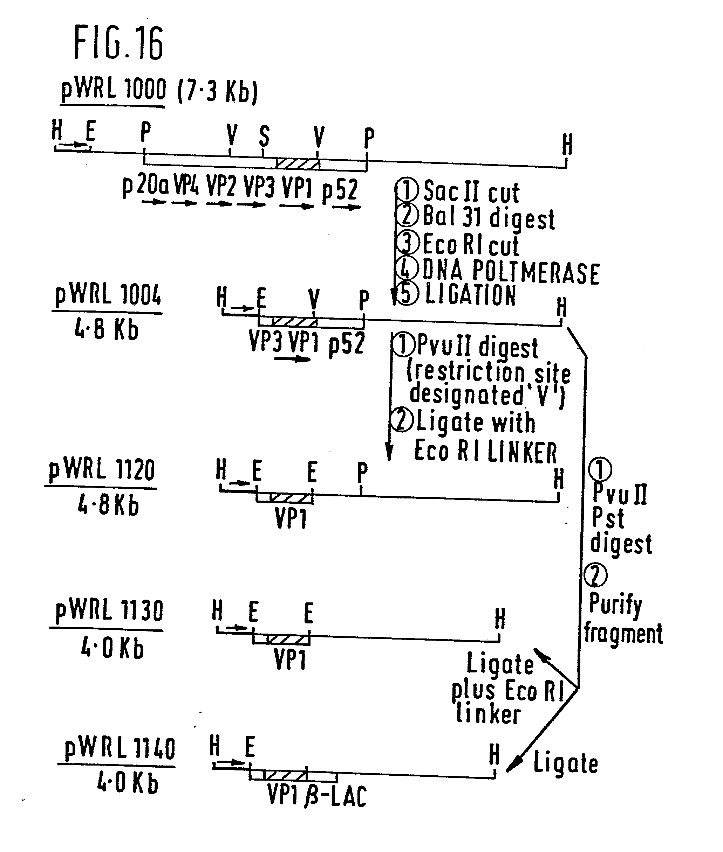

2 µg of pWRL 1004 was digested with 6 units if each Pvu II and Pst I and the 4.0 Kb fragment isolated from an agarose gel was described previously. Half of this DNA competent cells of E. coli (resulting in a plasmid pWRL 1140) while the other half was ligated in the presence of an EcoR1 linker before being used to transform E. coli. A plasmid of this latter type was found to have an extra EcoR1 site introduced and is designated pWRL 1130 (figure 16).

Results

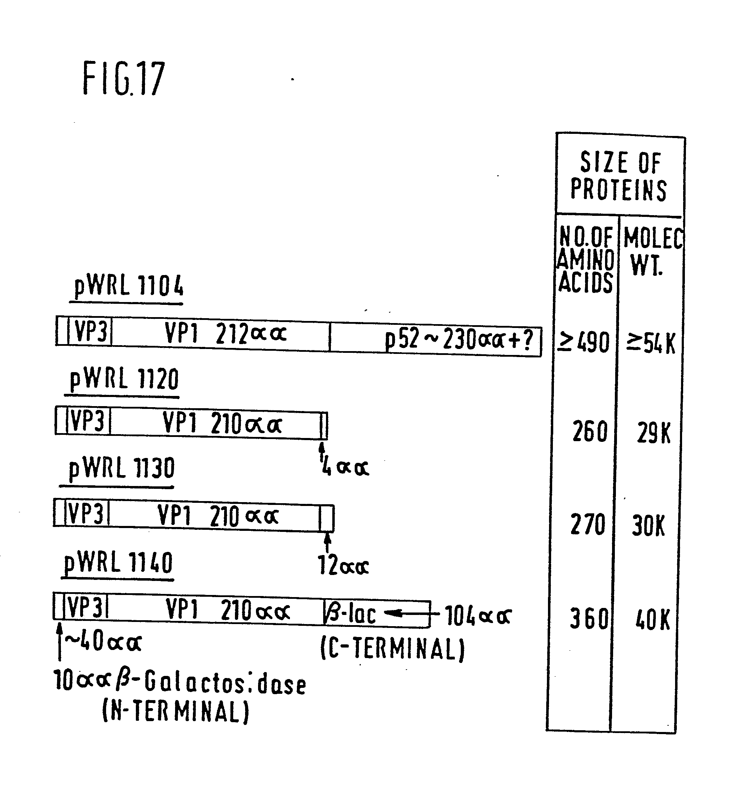

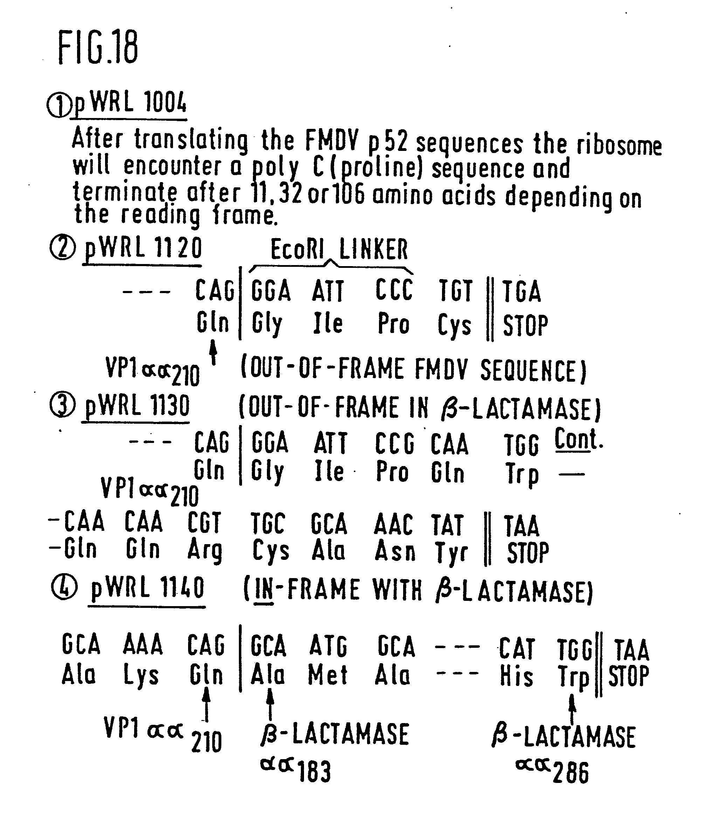

The molecular weights and C-terminal sequences of the predominant plasmid-encoded polypeptides synthesized by pWRL 1004, 1120, 1130 and 1140 are shown in figures 17 and 18. The large portion synthesized by pWRL 1004 contained sequences from the p52 polypeptide of FMDV and is unstable. The smaller proteins made in pWRL 1120 and 1130 contain primarily in $VP_1$ sequences and appeared stable in "Maxicells". The 40,000 dalton protein made by pWRL 1140 is a fusion product containing the C-terminal 104 amino acids of β-lactamase in addition to FMDV sequences and resulted in a stabilization of the polypeptide. All four of the polypeptides described above were synthesized in increased amounts when the bacteria are grown in the presence of 30μg/ml IPTG showing that the protein were produced under the control of the lac operon. The size of the protein produced was the same as what had been predicted.

Nucleotide sequence analysis

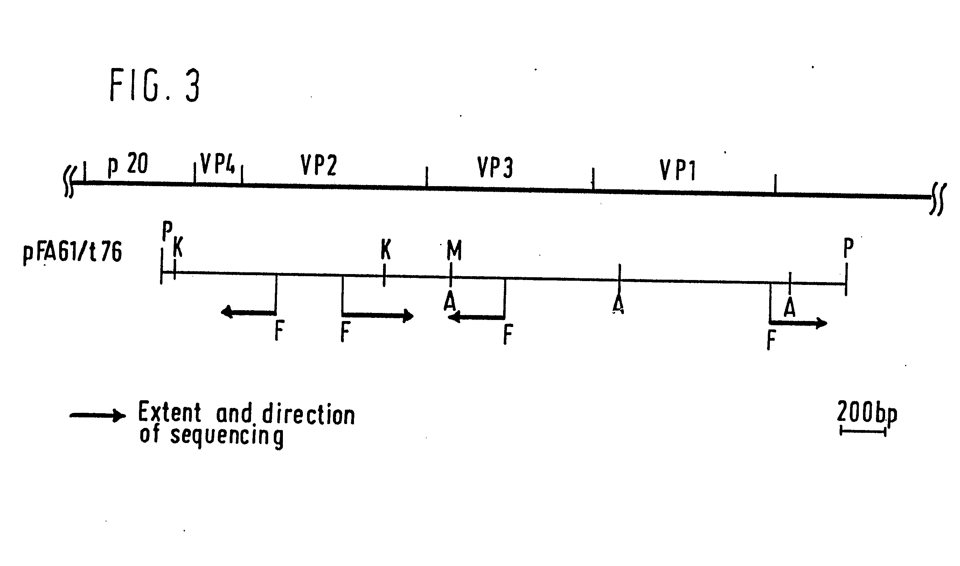

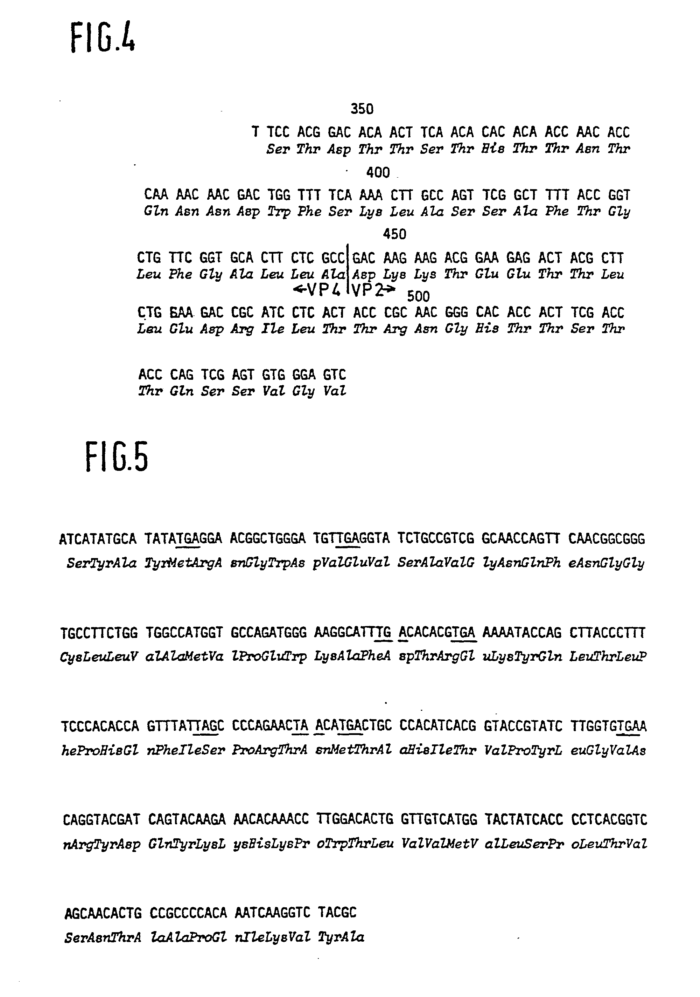

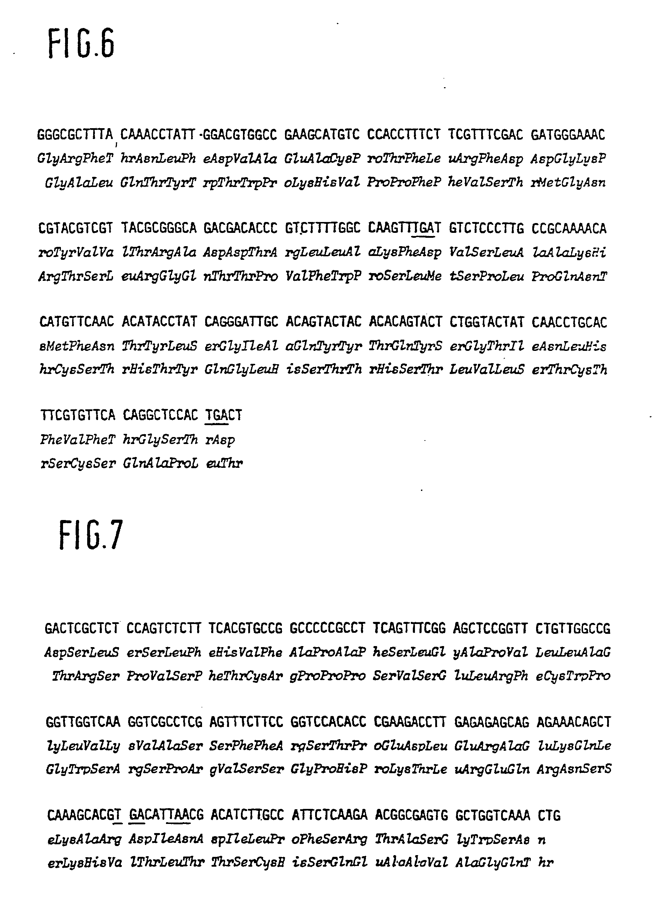

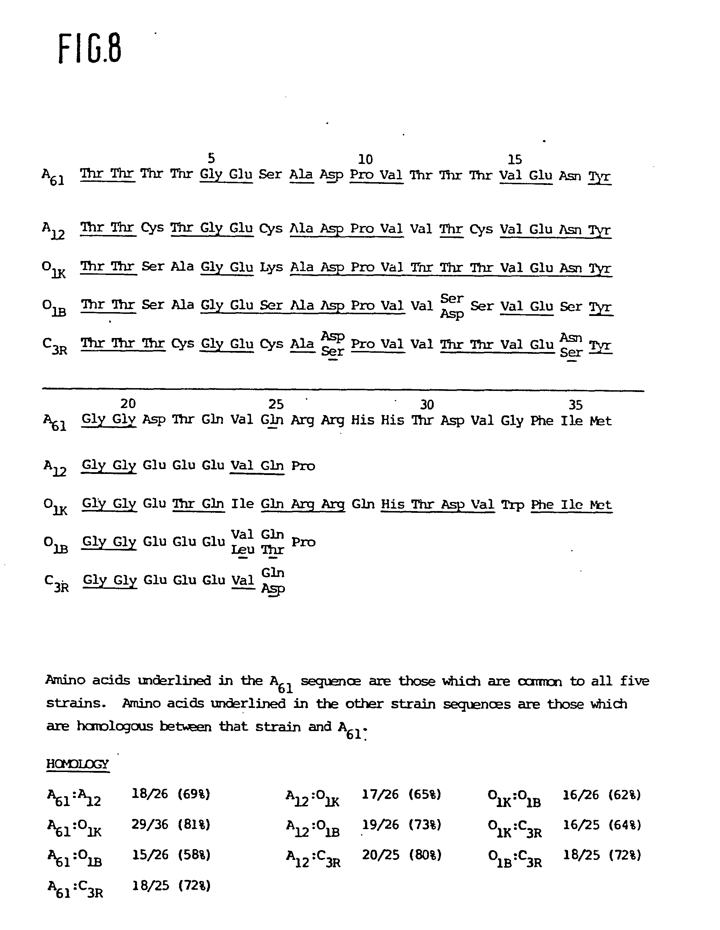

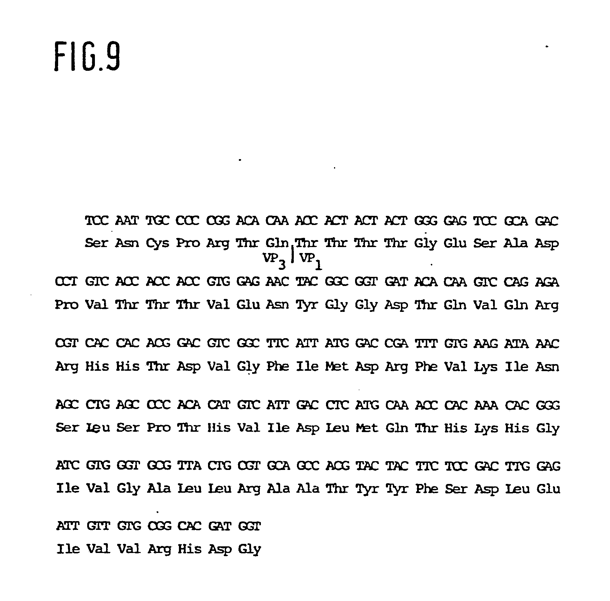

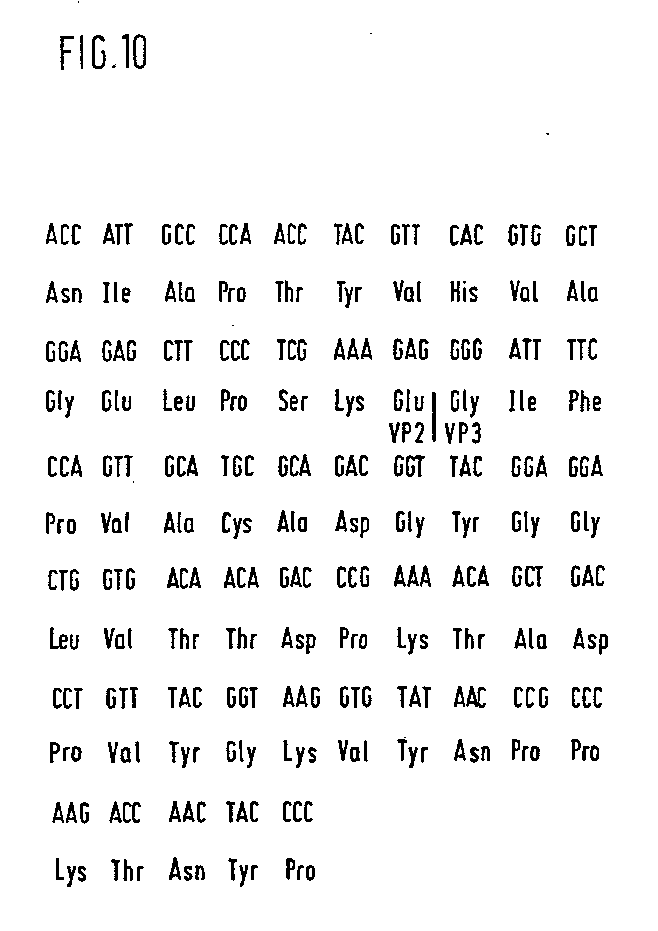

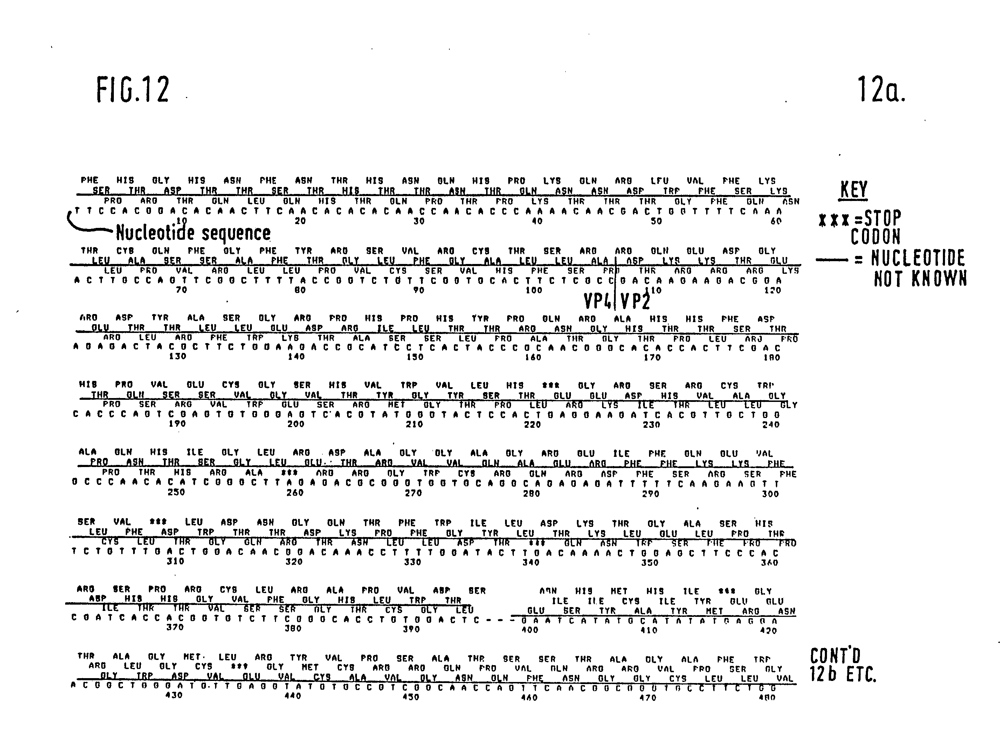

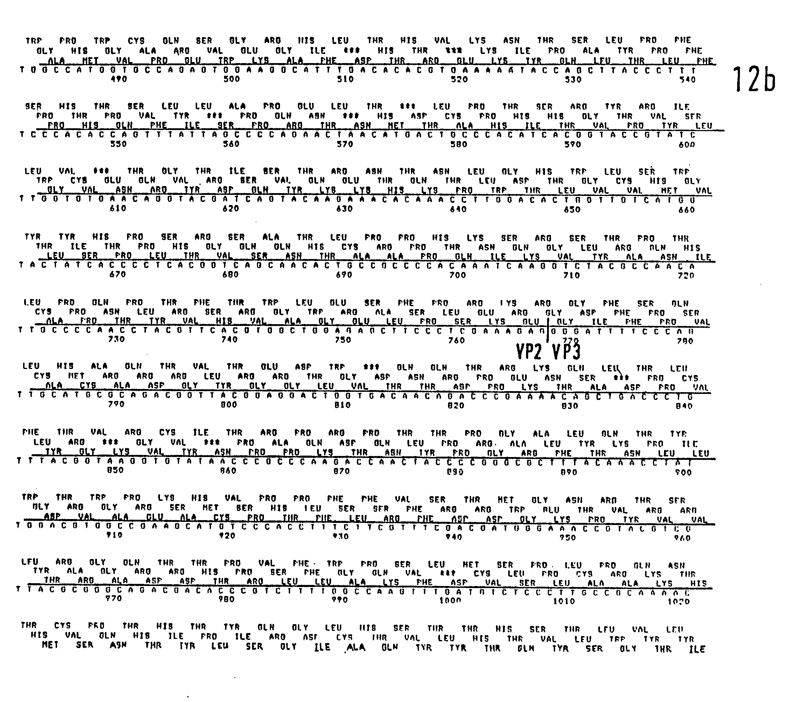

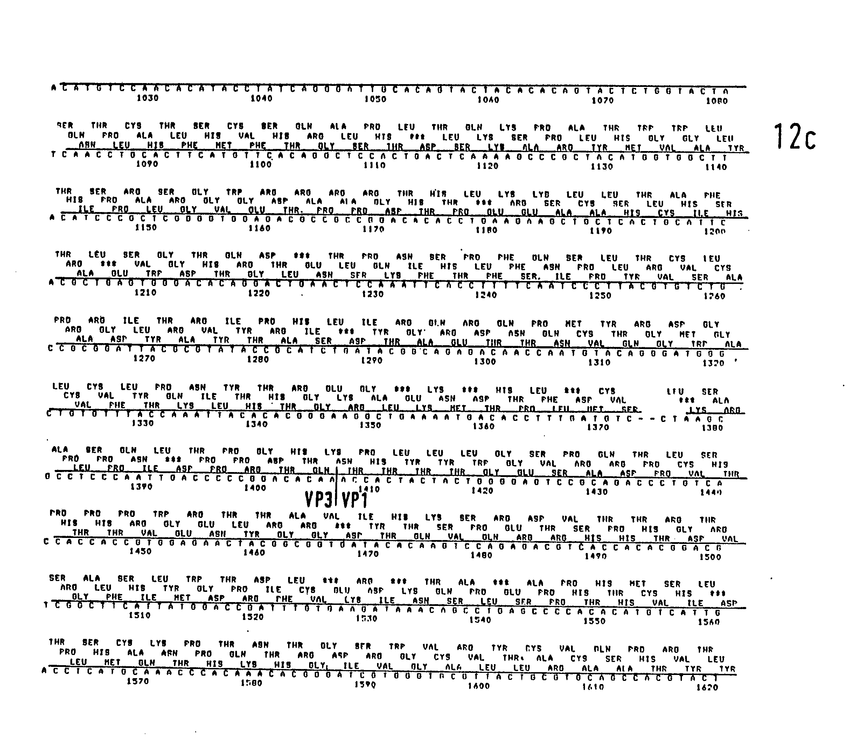

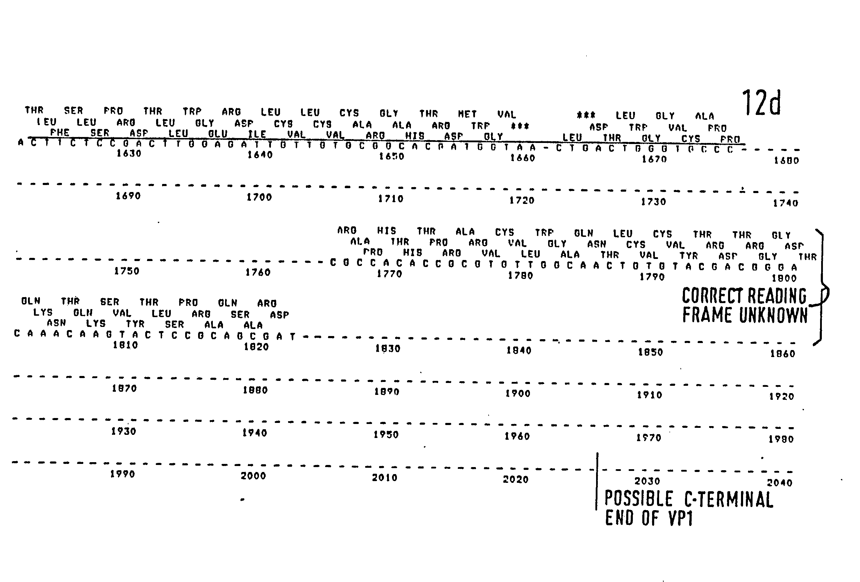

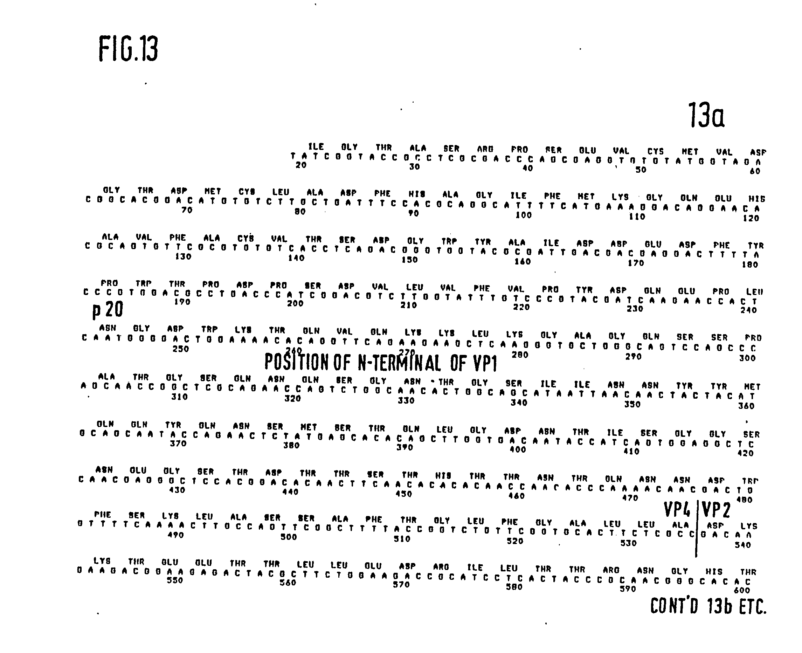

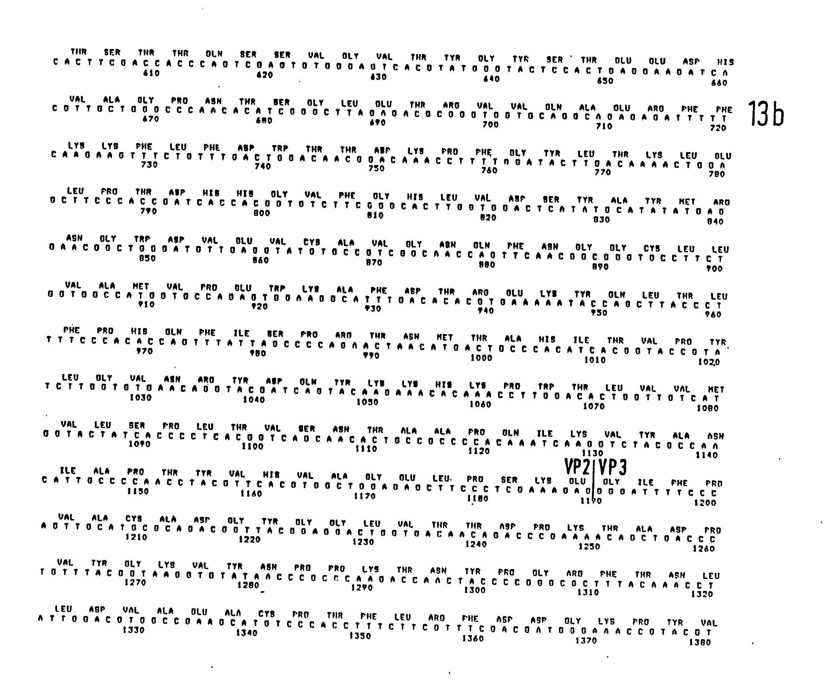

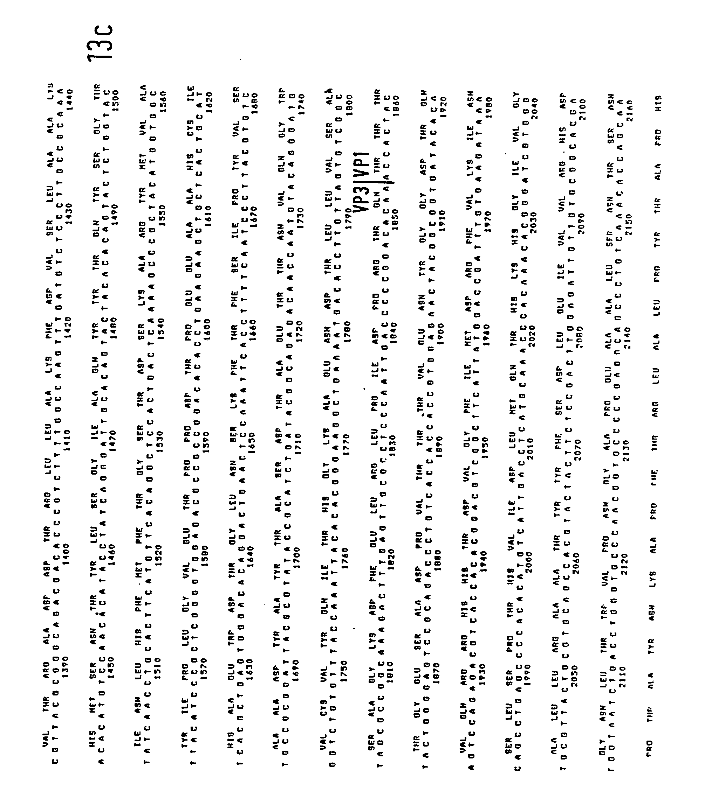

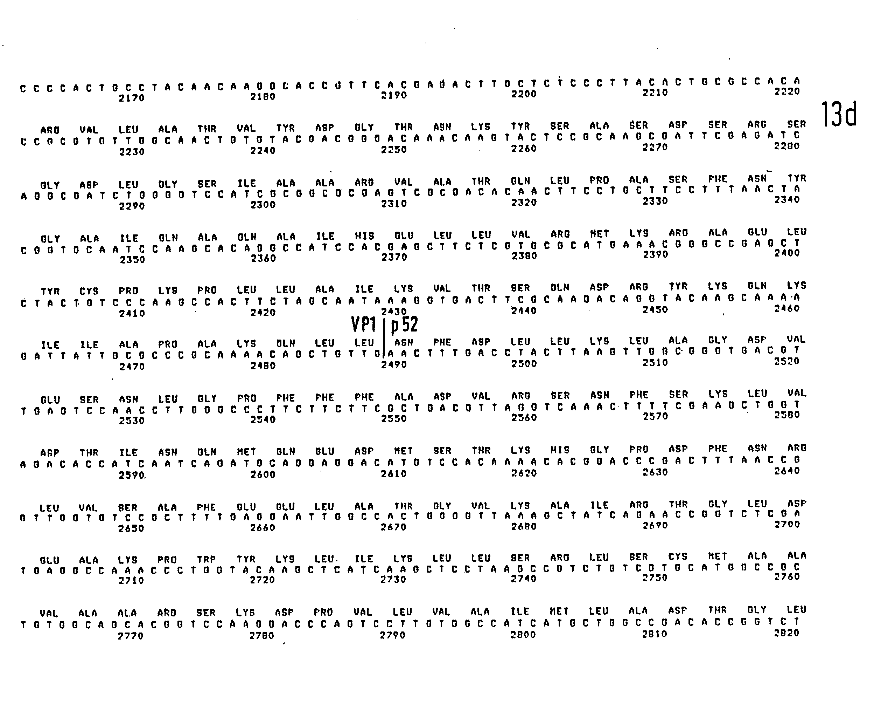

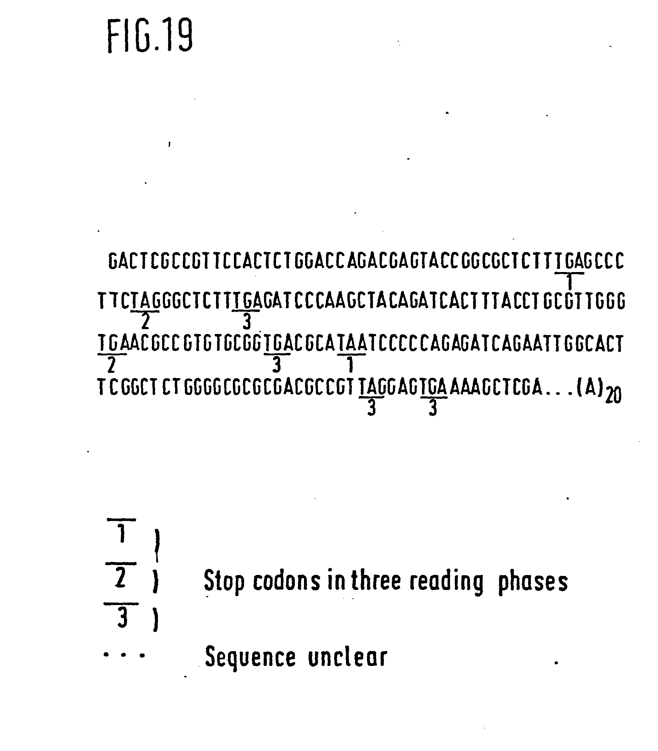

DNA fragments were prepared from the recombinant plasmids by digestion with appropriate restriction endonucleases. The fragments were appropriately end-labelled and the nucleotide sequences determined according to the procedures described by A Maxam and W Gilbert 1977 loc cit. From this details of the structural gene region of FMDV RNA have been determined, as shown in Figure 3. Figures 4 to 10, 12 and 13 show details of the DNA and amino acid sequence in the region of the $VP_4/VP_2$ junction and various other parts of the structural proteins. Figure 9 shows details of the DNA sequence of the C-terminal of $VP_3$ and N-terminal of $VP_1$. Figure 19 shows the nucleotide sequence of the 3' end of FMDV RNA which does not code for a protein.

Example 2

The processes used in Example 1 for preparing recombinant plasmids were repeated except that the strain of FMDV used was $O_1$BFS (also available from Animal Virus Research Institute).

Results

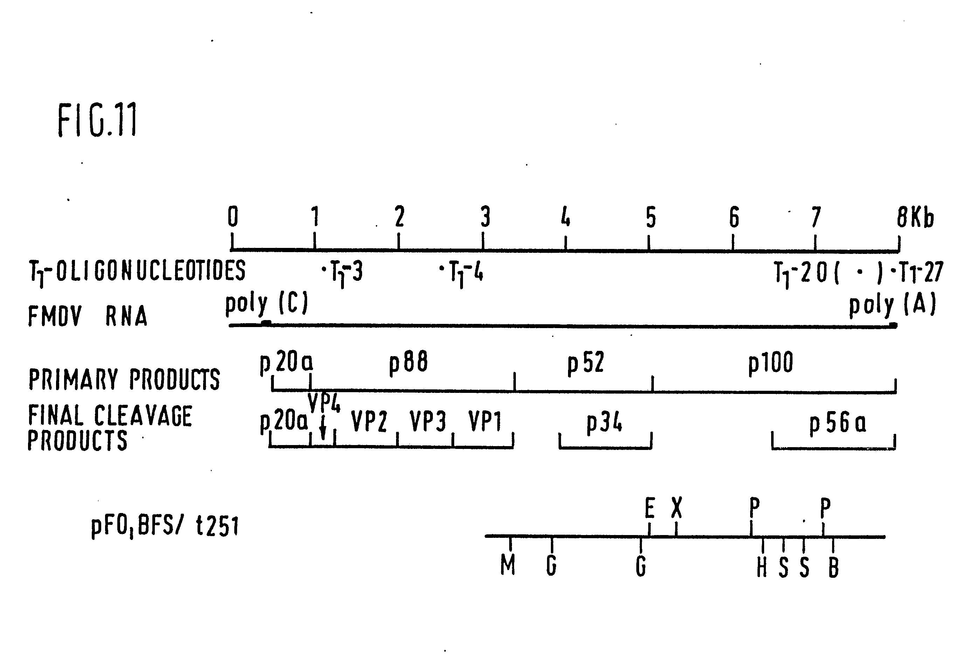

The physical map of a recombinant plasmid pF$O_1$BFS/t251 and its alignment and correspondence with the genes of FMDV, as deduced by application of the above methods, is shown in Figure 11.

Claims

1. A DNA molecule comprising a nucleotide sequence substantially corresponding to all or a portion of foot and mouth disease virus RNA (hereinafter referred to as FMDV).

2. A DNA molecule as claimed in claim 1 comprising a nucleotide sequence substantially coding for at least one protein of FMDV.

3. A DNA molecule as claimed in claim 2 wherein the nucleotide sequence substantially codes for a precursor of FMDV capsid proteins.

4. A DNA molecule as claimed in claim 2 wherein the nucleotide sequence substantially codes for FMDV protein p88.

5. A DNA molecule as claimed in claim 2 wherein the nucleotide sequence substantially codes for FMDV proteins $VP_1$, $VP_2$, $VP_3$ and $VP_4$.

6. A DNA molecule as claimed in claim 2 wherein the nucleotide sequence substantially codes for FMDV proteins $VP_4$, $VP_2$, $VP_3$ and $VP_1$ contiguously.

7. A DNA molecule as claimed in claim 2 wherein the nucleotide sequence substantially codes for FMDV protein $VP_1$ contiguous with the whole or part of any one of proteins $VP_2$, $VP_3$ and $VP_4$.

8. A DNA molecule as claimed in claim 2 wherein the nucleotide sequence substantially codes only for FMDV protein $VP_1$ alone or contiguous with the whole or part of protein $VP_3$.

9. A DNA molecule as claimed in claim 2 wherein the nucleotide sequence substantially codes for FMDV protein $VP_1$.

10. A DNA molecule as claimed in claim 2 wherein the nucleotide sequence substantially codes for FMDV protein $VP_2$.

11. A DNA molecule as claimed in claim 2 wherein the nucleotide sequence substantially codes for FMDV protein $VP_3$.

12. A DNA molecule as claimed in claim 2 wherein the nucleotide sequence substantially codes for FMDV protein $VP_4$.

13. A DNA molecule as claimed in claim 2 wherein the nucleotide sequence substantially codes for all or a portion of at least two FMDV proteins derived from one or more different variants of FMDV.

14. A DNA molecule as claimed in claim 13 wherein the FMDV proteins or portions thereof are $VP_1$ proteins from different variants of FMDV.

15. A DNA molecule as claimed in claim 9 wherein the nucleotide sequence is substantially as shown in Figure 13.

16. A DNA molecule as claimed in claim 10 wherein the nucleotide sequence is substantially as shown in Figure 13.

17. A DNA molecule as claimed in claim 11 wherein the nucleotide sequence is substantially as shown in Figure 13.

18. A DNA molecule as claimed in claim 12 wherein the nucleotide sequence is substantially as shown in Figure 13.

19. A DNA molecule as claimed in any one of claims 1 to 18 wherein the nucleotide sequence codes for a biologically functional fragment.

20. A DNA molecule as claimed in any one of claims 1 to 19 wherein the nucleotide sequence corresponds to RNA or codes for a protein of FMDV serotype A or serotype O.

21. A DNA molecule as claimed in claim 20 wherein the nucleotide sequence codes for a protein of FMDV serotype A, sub-type A10 strain 61.

22. A recombinant DNA molecule comprising an operon having initiator sequences, terminator sequences, as hereinbefore defined, and a nucleotide sequence substantially coding for all or a part of at least one protein of FMDV, the nucleotide sequence being located between the initator sequences and the terminator sequences of the operon.

23. A recombinant DNA molecule as claimed in claim 22 wherein the nucleotide sequence comprises a DNA molecule as claimed in any one of claims 2 to 21.

24. A recombinant DNA cloning vehicle capable of expressing all or part of a protein of FMDV, comprising a recombinant DNA molecule as claimed in claim 22 or claim 23.

25. A host cell containing at least one recombinant cloning vehicle as claimed in claim 24.

26. An antigen produced by a host cell transformed with recombinant DNA cloning vehicle according to claim 24 for stimulating the production of antibodies against FMDV in a mammal.

27. A vaccine for stimulating the production of antibodies against FMDV in a mammal comprising at least one protein displaying FMDV immunogenicity produced by a host cell as claimed in claim 25 together with a veterinarily acceptable carrier therefor.

28. A method of preparing a DNA molecule as claimed in any one of claims 1 to 21 comprising:

(a) isolating FMDV single stranded RNA;

(b) preparing a first single strand of DNA complementary to the single stranded RNA;

(c) preparing a second strand of DNA complementary to and hydrogen bonded to the first DNA strand.

29. A method of preparing a recombinant DNA cloning vehicle as claimed in claim 24 comprising:

(a) cleaving a cloning vehicle to produce free ends;

(b) inserting the DNA molecule as prepared in claim 28 in a location intermediate but not necessarily adjacent to the free ends of the cloning vehicle;

(c) joining the DNA molecule to the ends of the cloning vehicle by methods known per se.

30. A method of preparing a host cell as claimed in claim 25comprising transforming a host cell with a recombinant DNA cloning vehicle as prepared in claim 29.

31. A method of mapping the recombinant DNA molecule as claimed in any one of claims 1 to 21 comprising:-

(a) digesting the DNA with restriction endonuclease enzymes and separating the products;

(b) adding labelled oligonucleotides of FMDV RNA produced by $T_1$ ribonuclease digestion;

(c) identifying the products of restriction endonuclease digestion to which the $T_1$ oligonucleotides have hybridized.

32. A DNA molecule as defined in any one of claims 1 to 21 prepared by the method as claimed in claim 28.

33. A recombinant DNA cloning vehicle as defined in claim 24 prepared by the method as claimed in claim 29.

34. A host cell as defined in claim 25 prepared by the method as claimed in claim 30.

FIG.1

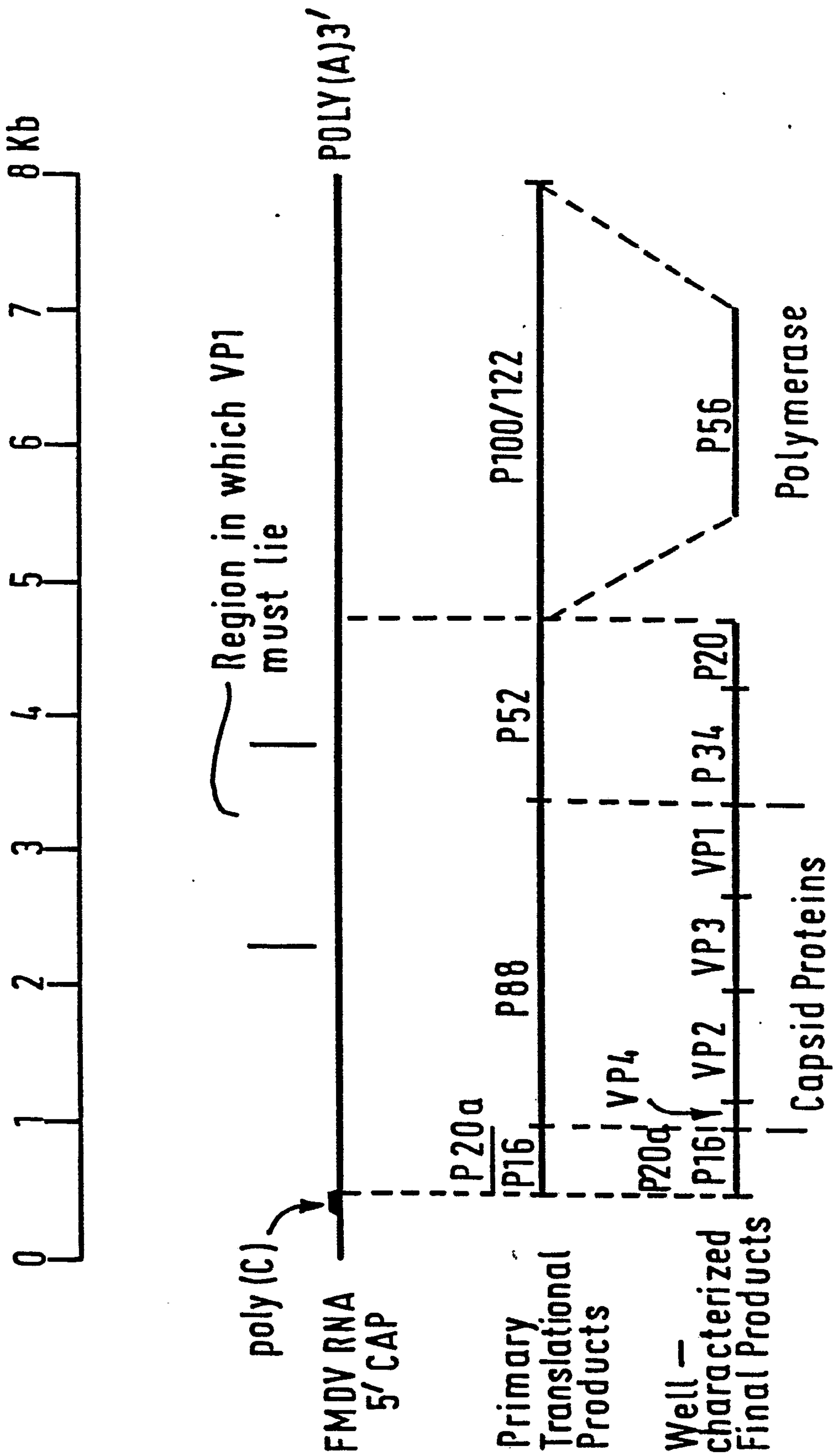
0
1
2
3
4
5
6
7
8 Kb
FMDV RNA
5' CAP
poly (C)
POLY(A)3'
Region in which VP1
must lie
Primary
Translational
Products
P20a
P16
P88
P52
P100/122
Well —
characterized
Final Products
P20a
P16
VP4
VP2
VP3
VP1
P34
P20
P56
Polymerase
Capsid Proteins

FIG.2

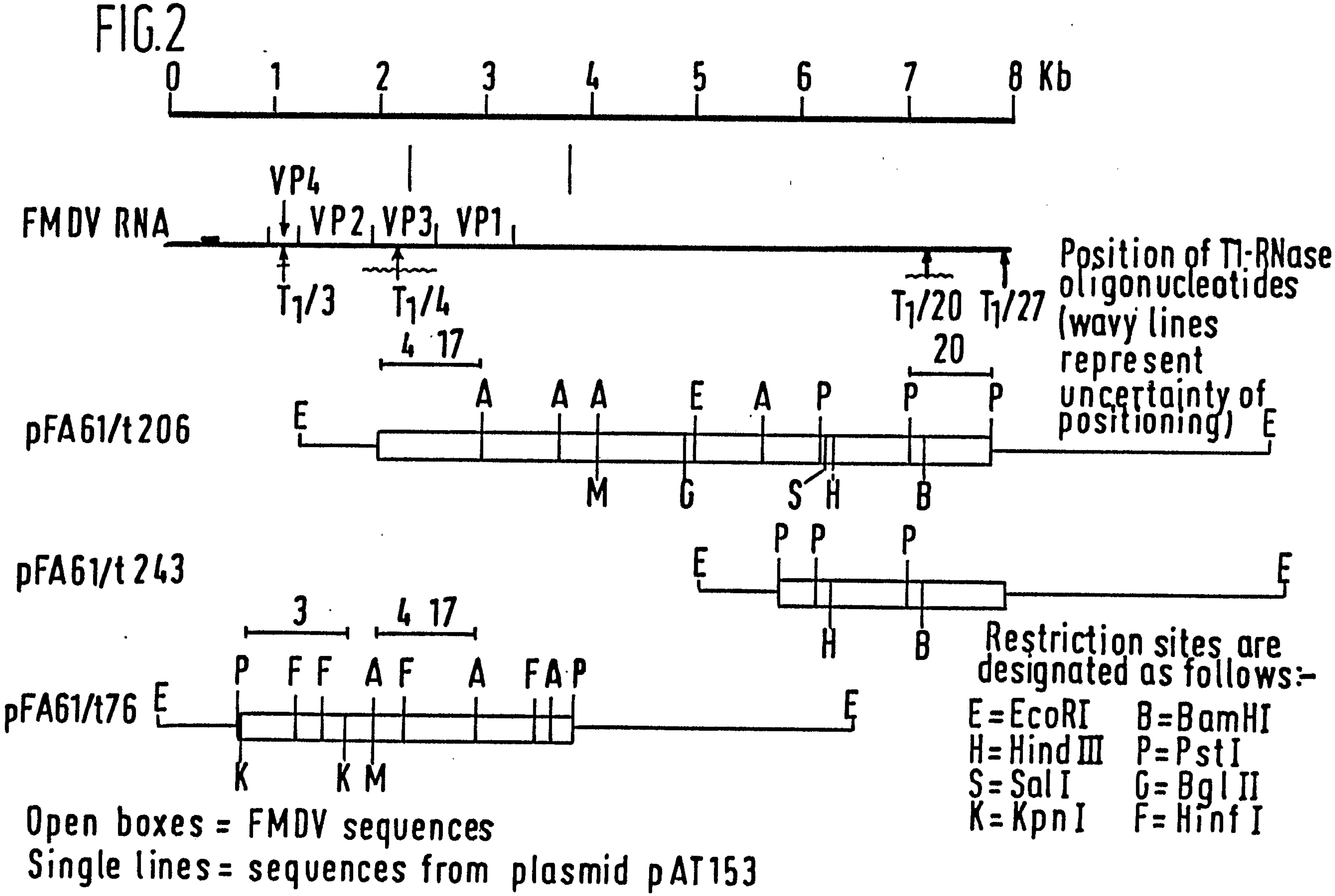


Open boxes = FMDV sequences
Single lines = sequences from plasmid pAT153

FIG. 3

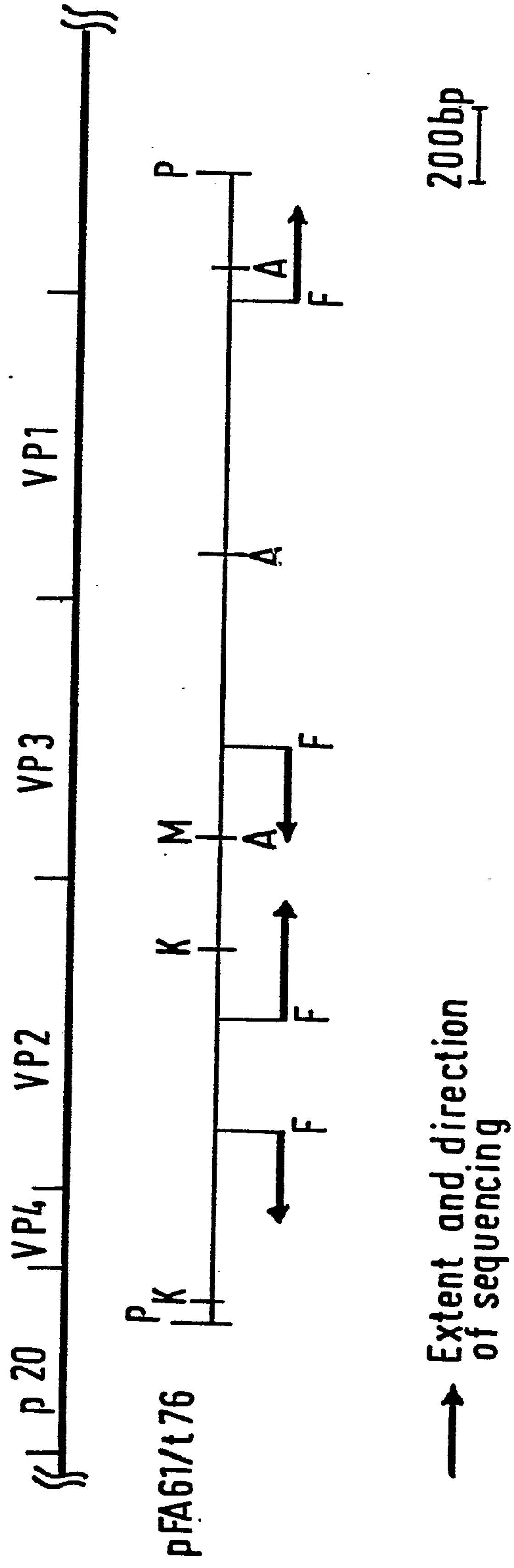
p 20
VP4
VP2
VP3
VP1
pFA61/t76
P
K
K
M
A
A
A
P
F
F
F
F
200bp
Extent and direction of sequencing

FIG.4

350

T TCC ACG GAC ACA ACT TCA ACA CAC ACA ACC AAC ACC
*Ser Thr Asp Thr Thr Ser Thr His Thr Thr Asn Thr*

400

CAA AAC AAC GAC TGG TTT TCA AAA CTT GCC AGT TCG GCT TTT ACC GGT
*Gln Asn Asn Asp Trp Phe Ser Lys Leu Ala Ser Ser Ala Phe Thr Gly*

450

CTG TTC GGT GCA CTT CTC GCC | GAC AAG AAG ACG GAA GAG ACT ACG CTT
*Leu Phe Gly Ala Leu Leu Ala | Asp Lys Lys Thr Glu Glu Thr Thr Leu*
←VP4 | VP2→ 500

CTG GAA GAC CGC ATC CTC ACT ACC CGC AAC GGG CAC ACC ACT TCG ACC
*Leu Glu Asp Arg Ile Leu Thr Thr Arg Asn Gly His Thr Thr Ser Thr*

ACC CAG TCG AGT GTG GGA GTC
*Thr Gln Ser Ser Val Gly Val*

FIG.5

ATCATATGCA TATATGAGGA ACGGCTGGGA TGTTGAGGTA TCTGCCGTCG GCAACCAGTT CAACGGCGGG
*SerTyrAla TyrMetArgA snGlyTrpAs pValGluVal SerAlaValG lyAsnGlnPh eAsnGlyGly*

TGCCTTCTGG TGGCCATGGT GCCAGATGGG AAGGCATTTG ACACACGTGA AAAATACCAG CTTACCCTTT
*CysLeuLeuV alAlaMetVa lProGluTrp LysAlaPheA spThrArgGl uLysTyrGln LeuThrLeuP*

TCCCACACCA GTTTATTAGC CCCAGAACTA ACATGACTGC CCACATCACG GTACCGTATC TTGGTGTGAA
*heProHisGl nPheIleSer ProArgThrA snMetThrAl aHisIleThr ValProTyrL euGlyValAs*

CAGGTACGAT CAGTACAAGA AACACAAACC TTGGACACTG GTTGTCATGG TACTATCACC CCTCACGGTC
*nArgTyrAsp GlnTyrLysL ysHisLysPr oTrpThrLeu ValValMetV alLeuSerPr oLeuThrVal*

AGCAACACTG CCGCCCCACA AATCAAGGTC TACGC
*SerAsnThrA laAlaProGl nIleLysVal TyrAla*

# FIG.6

GGGCGCTTTA CAAACCTATT GGACGTGGCC GAAGCATGTC CCACCTTTCT TCGTTTCGAC GATGGGAAAC
*GlyArgPheT hrAsnLeuPh eAspValAla GluAlaCysP roThrPheLe uArgPheAsp AspGlyLysP*
*GlyAlaLeu GlnThrTyrT rpThrTrpPr oLysHisVal ProProPheP heValSerTh rMetGlyAsn*

CGTACGTCGT TACGCGGGCA GACGACACCC GTCTTTTGGC CAAGTTTGAT GTCTCCCTTG CCGCAAAACA
*roTyrValVa lThrArgAla AspAspThrA rgLeuLeuAl aLysPheAsp ValSerLeuA laAlaLysHi*
*ArgThrSerL euArgGlyGl nThrThrPro ValPheTrpP roSerLeuMe tSerProLeu ProGlnAsnT*

CATGTTCAAC ACATACCTAT CAGGGATTGC ACAGTACTAC ACACAGTACT CTGGTACTAT CAACCTGCAC
*sMetPheAsn ThrTyrLeuS erGlyIleAl aGlnTyrTyr ThrGlnTyrS erGlyThrIl eAsnLeuHis*
*hrCysSerTh rHisThrTyr GlnGlyLeuH isSerThrTh rHisSerThr LeuValLeuS erThrCysTh*

TTCGTGTTCA CAGGCTCCAC TGACT
*PheValPheT hrGlySerTh rAsp*
*rSerCysSer GlnAlaProL euThr*

# FIG.7

GACTCGCTCT CCAGTCTCTT TCACGTGCCG GCCCCCGCCT TCAGTTTCGG AGCTCCGGTT CTGTTGGCCG
*AspSerLeuS erSerLeuPh eHisValPhe AlaProAlaP heSerLeuGl yAlaProVal LeuLeuAlaG*
*ThrArgSer ProValSerP heThrCysAr gProProPro SerValSerG luLeuArgPh eCysTrpPro*

GGTTGGTCAA GGTCGCCTCG AGTTTCTTCC GGTCCACACC CGAAGACCTT GAGAGAGCAG AGAAACAGCT
*lyLeuValLy sValAlaSer SerPhePheA rgSerThrPr oGluAspLeu GluArgAlaG luLysGlnLe*
*GlyTrpSerA rgSerProAr gValSerSer GlyProHisP roLysThrLe uArgGluGln ArgAsnSerS*

CAAAGCACGT GACATTAACG ACATCTTGCC ATTCTCAAGA ACGGCGAGTG GCTGGTCAAA CTG
*eLysAlaArg AspIleAsnA spIleLeuPr oPheSerArg ThrAlaSerG lyTrpSerAs n*
*erLysHisVa lThrLeuThr ThrSerCysH isSerGlnGl uAlaAlaVal AlaGlyGlnT hr*

# FIG.8

| | 1 | 2 | 3 | 4 | 5 | 6 | 7 | 8 | 9 | 10 | 11 | 12 | 13 | 14 | 15 | 16 | 17 | 18 |
|---|---|---|---|---|---|---|---|---|---|---|---|---|---|---|---|---|---|---|
| $A_{61}$ | Thr | Thr | Thr | Thr | Gly | Glu | Ser | Ala | Asp | Pro | Val | Thr | Thr | Thr | Val | Glu | Asn | Tyr |
| $A_{12}$ | Thr | Thr | Cys | Thr | Gly | Glu | Cys | Ala | Asp | Pro | Val | Val | Thr | Cys | Val | Glu | Asn | Tyr |
| $O_{1K}$ | Thr | Thr | Ser | Ala | Gly | Glu | Lys | Ala | Asp | Pro | Val | Thr | Thr | Thr | Val | Glu | Asn | Tyr |
| $O_{1B}$ | Thr | Thr | Ser | Ala | Gly | Glu | Ser | Ala | Asp | Pro | Val | Val | Ser/Asp | Ser | Val | Glu | Ser | Tyr |
| $C_{3R}$ | Thr | Thr | Thr | Cys | Gly | Glu | Cys | Ala | Asp/Ser | Pro | Val | Val | Thr | Thr | Val | Glu | Asn/Ser | Tyr |

| | 19 | 20 | 21 | 22 | 23 | 24 | 25 | 26 | 27 | 28 | 29 | 30 | 31 | 32 | 33 | 34 | 35 | 36 |
|---|---|---|---|---|---|---|---|---|---|---|---|---|---|---|---|---|---|---|
| $A_{61}$ | Gly | Gly | Asp | Thr | Gln | Val | Gln | Arg | Arg | His | His | Thr | Asp | Val | Gly | Phe | Ile | Met |
| $A_{12}$ | Gly | Gly | Glu | Glu | Glu | Val | Gln | Pro | | | | | | | | | | |
| $O_{1K}$ | Gly | Gly | Glu | Thr | Gln | Ile | Gln | Arg | Arg | Gln | His | Thr | Asp | Val | Trp | Phe | Ile | Met |
| $O_{1B}$ | Gly | Gly | Glu | Glu | Glu | Val/Leu | Gln/Thr | Pro | | | | | | | | | | |
| $C_{3R}$ | Gly | Gly | Glu | Glu | Glu | Val | Gln/Asp | | | | | | | | | | | |

Amino acids underlined in the $A_{61}$ sequence are those which are common to all five strains. Amino acids underlined in the other strain sequences are those which are homologous between that strain and $A_{61}$.

HOMOLOGY

| | | | | | |
|---|---|---|---|---|---|
| $A_{61}:A_{12}$ | 18/26 (69%) | $A_{12}:O_{1K}$ | 17/26 (65%) | $O_{1K}:O_{1B}$ | 16/26 (62%) |
| $A_{61}:O_{1K}$ | 29/36 (81%) | $A_{12}:O_{1B}$ | 19/26 (73%) | $O_{1K}:C_{3R}$ | 16/25 (64%) |
| $A_{61}:O_{1B}$ | 15/26 (58%) | $A_{12}:C_{3R}$ | 20/25 (80%) | $O_{1B}:C_{3R}$ | 18/25 (72%) |
| $A_{61}:C_{3R}$ | 18/25 (72%) | | | | |

FIG.9

TCC AAT TGC CCC CGG ACA CAA ACC ACT ACT ACT GGG GAG TCC GCA GAC
Ser Asn Cys Pro Arg Thr Gln | Thr Thr Thr Thr Gly Glu Ser Ala Asp
$VP_3$ | $VP_1$

CCT GTC ACC ACC ACC GTG GAG AAC TAC GGC GGT GAT ACA CAA GTC CAG AGA
Pro Val Thr Thr Thr Val Glu Asn Tyr Gly Gly Asp Thr Gln Val Gln Arg

CGT CAC CAC ACG GAC GTC GGC TTC ATT ATG GAC CGA TTT GTG AAG ATA AAC
Arg His His Thr Asp Val Gly Phe Ile Met Asp Arg Phe Val Lys Ile Asn

AGC CTG AGC CCC ACA CAT GTC ATT GAC CTC ATG CAA ACC CAC AAA CAC GGG
Ser Leu Ser Pro Thr His Val Ile Asp Leu Met Gln Thr His Lys His Gly

ATC GTG GGT GCG TTA CTG CGT GCA GCC ACG TAC TAC TTC TCC GAC TTG GAG
Ile Val Gly Ala Leu Leu Arg Ala Ala Thr Tyr Tyr Phe Ser Asp Leu Glu

ATT GTT GTG CGG CAC GAT GGT
Ile Val Val Arg His Asp Gly

FIG.10

| | | | | | | | | | |
|---|---|---|---|---|---|---|---|---|---|
| ACC | ATT | GCC | CCA | ACC | TAC | GTT | CAC | GTG | GCT |
| Asn | Ile | Ala | Pro | Thr | Tyr | Val | His | Val | Ala |
| GGA | GAG | CTT | CCC | TCG | AAA | GAG | GGG | ATT | TTC |
| Gly | Glu | Leu | Pro | Ser | Lys | Glu \| VP2 | Gly \| VP3 | Ile | Phe |
| CCA | GTT | GCA | TGC | GCA | GAC | GGT | TAC | GGA | GGA |
| Pro | Val | Ala | Cys | Ala | Asp | Gly | Tyr | Gly | Gly |
| CTG | GTG | ACA | ACA | GAC | CCG | AAA | ACA | GCT | GAC |
| Leu | Val | Thr | Thr | Asp | Pro | Lys | Thr | Ala | Asp |
| CCT | GTT | TAC | GGT | AAG | GTG | TAT | AAC | CCG | CCC |
| Pro | Val | Tyr | Gly | Lys | Val | Tyr | Asn | Pro | Pro |
| AAG | ACC | AAC | TAC | CCC | | | | | |
| Lys | Thr | Asn | Tyr | Pro | | | | | |

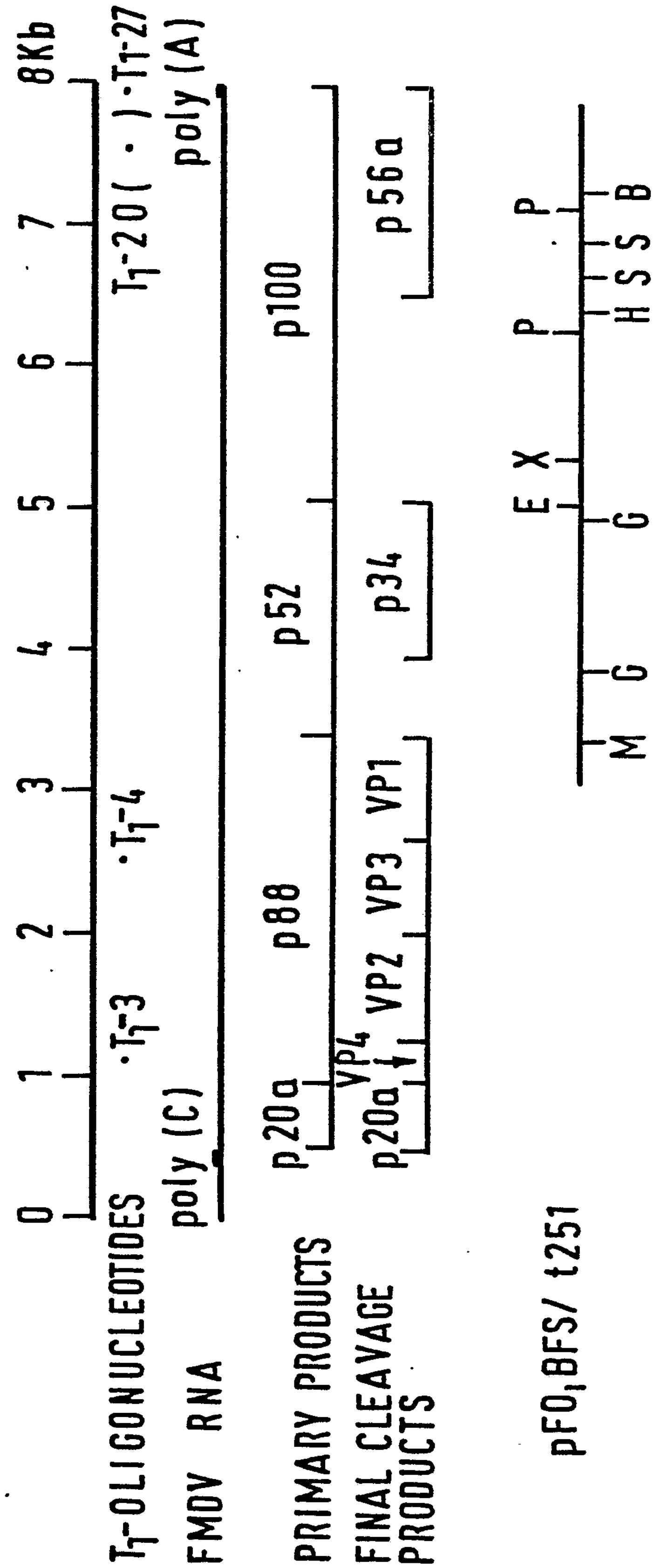
T1-OLIGONUCLEOTIDES
FMDV RNA
PRIMARY PRODUCTS
FINAL CLEAVAGE PRODUCTS
pFO1BFS/ t251
0
1
2
3
4
5
6
7
8Kb
·T1-3
·T1-4
T1-20( · )·T1-27
poly (C)
poly (A)
p20a
p88
p52
p100
p20a
VP4
VP2
VP3
VP1
p34
p56a
M
G
G
E
X
P
H
S
S
P
B

FIG.11

FIG.12

12a.

```
PHE HIS GLY HIS ASN PHE ASN THR HIS ASN GLN HIS PRO LYS GLN ARG LEU VAL PHE LYS
 SER THR ASP THR THR SER THR HIS THR THR ASN THR GLN ASN ASN ASP TRP PHE SER LYS
  PRO ARG THR GLN LEU GLN HIS THR GLN PRO THR PRO LYS THR THR THR GLY PHE GLN ASN
T T C C A C G G A C A C A A C T T C A A C A C A C A C A A C C A A C A C C C A A A A C A A C G A C T G G T T T T C A A A
                 10                  20                  30                  40                  50                  60
Nucleotide sequence

THR CYS GLN PHE GLY PHE TYR ARG SER VAL ARG CYS THR SER ARG ARG GLN GLU ASP GLY
 LEU ALA SER SER ALA PHE THR GLY LEU PHE GLY ALA LEU LEU ALA | ASP LYS LYS THR GLU
  LEU PRO VAL ARG LEU LEU PRO VAL CYS SER VAL HIS PHE SER PRO | THR ARG ARG ARG LYS
A C T T G C C A G T T C G G C T T T T A C C G G T C T G T T C G G T G C A C T T C T C G C C | G A C A A G A A G A C G G A
                 70                  80                  90                  100                 110                 120
                                                                          VP4|VP2

ARG ASP TYR ALA SER GLY ARG PRO HIS PRO HIS TYR PRO GLN ARG ALA HIS HIS PHE ASP
 GLU THR THR LEU LEU GLU ASP ARG ILE LEU THR THR ARG ASN GLY HIS THR THR SER THR
  ARG LEU ARG PHE TRP LYS THR ALA SER SER LEU PRO ALA THR GLY THR PRO LEU ARG PRO
A G A G A C T A C G C T T C T G G A A G A C C G C A T C C T C A C T A C C C G C A A C G G G C A C A C C A C T T C G A C
                 130                 140                 150                 160                 170                 180

HIS PRO VAL GLU CYS GLY SER HIS VAL TRP VAL LEU HIS *** GLY ARG SER ARG CYS TRP
 THR GLN SER SER VAL GLY VAL THR TYR GLY TYR SER THR GLU GLU ASP HIS VAL ALA GLY
  PRO SER ARG VAL TRP GLU SER ARG MET GLY THR PRO LEU ARG LYS ILE THR LEU LEU GLY
C A C C C A G T C G A G T G T G G G A G T C A C G T A T G G G T A C T C C A C T G A G G A A G A T C A C G T T G C T G G
                 190                 200                 210                 220                 230                 240

ALA GLN HIS ILE GLY LEU ARG ASP ALA GLY GLY ALA GLY ARG GLU ILE PHE GLN GLU VAL
 PRO ASN THR SER GLY LEU GLU THR ARG VAL VAL GLN ALA GLU ARG PHE PHE LYS LYS PHE
  PRO THR HIS ARG ALA *** ARG ARG GLY TRP CYS ARG GLN ARG ASP PHE SER ARG SER PHE
G C C C A A C A C A T C G G G C T T A G A G A C G C G G G T G G T G C A G G C A G A G A G A T T T T T C A A G A A G T T
                 250                 260                 270                 280                 290                 300

SER VAL *** LEU ASP ASN GLY GLN THR PHE TRP ILE LEU ASP LYS THR GLY ALA SER HIS
 LEU PHE ASP TRP THR THR ASP LYS PRO PHE GLY TYR LEU THR LYS LEU GLU LEU PRO THR
  CYS LEU THR GLY GLN ARG THR ASN LEU LEU ASP THR *** GLN ASN TRP SER PHE PRO PRO
T C T G T T T G A C T G G A C A A C G G A C A A A C C T T T T G G A T A C T T G A C A A A C T G G A G C T T C C C A C
                 310                 320                 330                 340                 350                 360

ARG SER PRO ARG CYS LEU ARG ALA PRO VAL ASP SER          ASN HIS MET HIS ILE *** GLY
 ASP HIS HIS GLY VAL PHE GLY HIS LEU TRP THR              ILE ILE CYS ILE TYR GLU GLU
  ILE THR THR VAL SER SER GLY THR CYS GLY LEU           GLU SER TYR ALA TYR MET ARG ASN
C G A T C A C C A C G G T G T C T T C G G G C A C C T G T G G A C T C - - - G A A T C A T A T G C A T A T A T G A G G A
                 370                 380                 390                 400                 410                 420

THR ALA GLY MET LEU ARG TYR VAL PRO SER ALA THR SER SER THR ALA GLY ALA PHE TRP
 ARG LEU GLY CYS *** GLY MET CYS ARG ARG GLN PRO VAL GLN ARG ARG VAL PRO SER GLY
  GLY TRP ASP VAL GLU VAL CYS ALA VAL GLY ASN GLN PHE ASN GLY GLY CYS LEU LEU VAL
A C G G C T G G G A T G T T G A G G T A T G T G C C G T C G G C A A C C A G T T C A A C G G C G G G T G C C T T C T G G
                 430                 440                 450                 460                 470                 480
```

**KEY**

*** = STOP CODON

— = NUCLEOTIDE NOT KNOWN

CONT'D 12b ETC.

12b

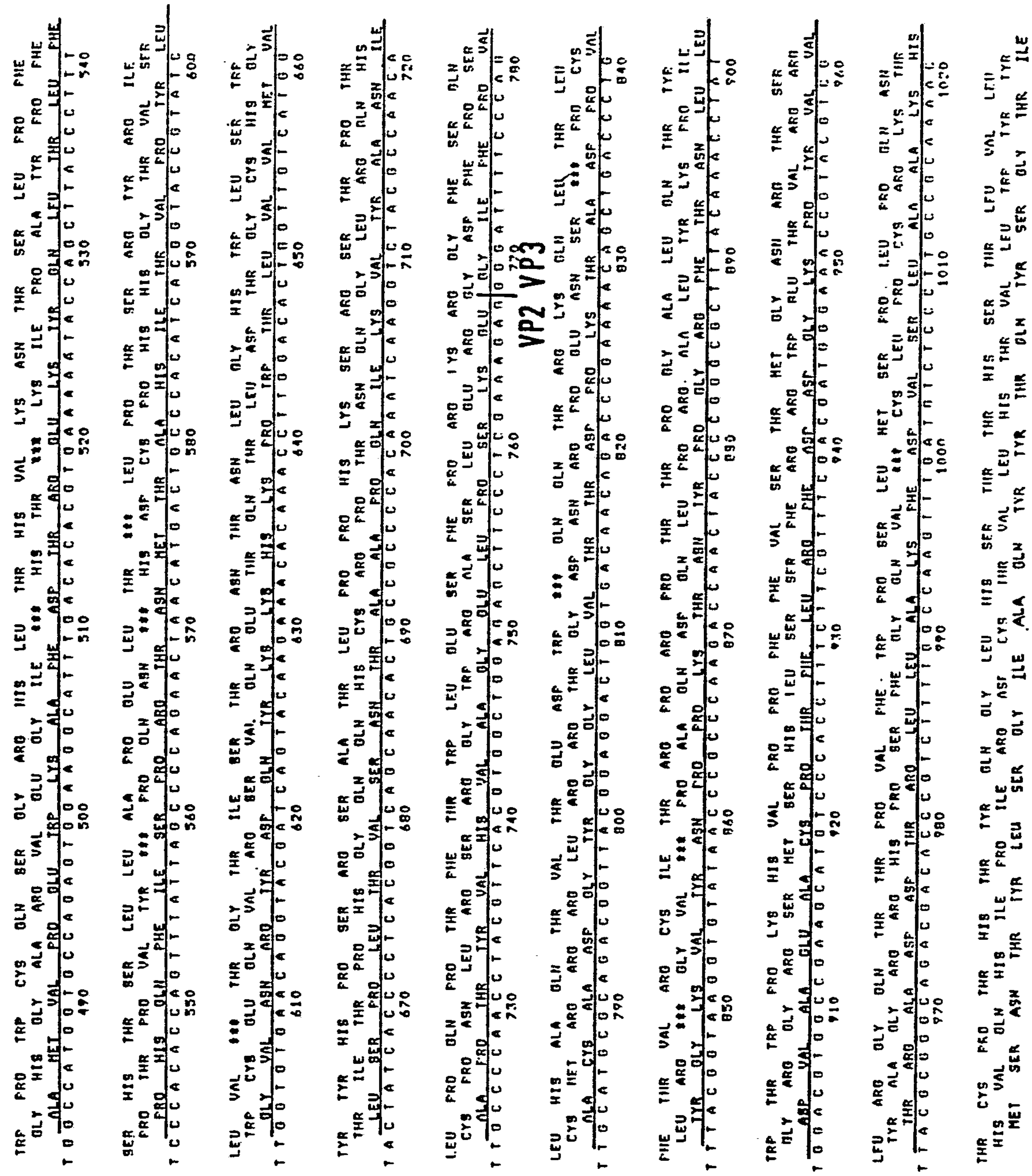

12c

```
ACATGTCCAACACATACCTATCAGGGATTGCACAGTACTACACACAGTACTCTGGTACTA
         1030      1040      1050      1060      1070      1080

SER THR CYS THR SER CYS SER GLN ALA PRO LEU THR GLN LYS PRO ALA THR TRP TRP LEU
 GLN PRO ALA LEU HIS VAL HIS ARG LEU HIS *** LEU LYS SER PRO LEU HIS GLY GLY LEU
  ASN LEU HIS PHE MET PHE THR GLY SER THR ASP SER LYS ALA ARG TYR MET VAL ALA TYR
TCAACCTGCACTTCATGTTCACAGGCTCCACTGACTCAAAAGCCCGCTACATGGTGGCTT
         1090      1100      1110      1120      1130      1140

THR SER ARG SER GLY TRP ARG ARG ARG ARG THR HIS LEU LYS LYS LEU LEU THR ALA PHE
 HIS PRO ALA ARG GLY GLY ASP ALA ALA GLY HIS THR *** ARG SER CYS SER LEU HIS SER
  ILE PRO LEU GLY VAL GLU THR. PRO PRO ASP THR PRO GLU GLU ALA ALA HIS CYS ILE HIS
ACATCCCGCTCGGGGTGGAGACGCCGCCGGACACACCTGAAGAAGCTGCTCACTGCATTC
         1150      1160      1170      1180      1190      1200

THR LEU SER GLY THR GLN ASP *** THR PRO ASN SER PRO PHE GLN SER LEU THR CYS LEU
 ARG *** VAL GLY HIS ARG THR GLU LEU GLN ILE HIS LEU PHE ASN PRO LEU ARG VAL CYS
  ALA GLU TRP ASP THR GLY LEU ASN SER LYS PHE THR PHE SER. ILE PRO TYR VAL SER ALA
ACGCTGAGTGGGACACAGGACTGAACTCCAAATTCACCTTTTCAATCCCTTACGTGTCTG
         1210      1220      1230      1240      1250      1260

PRO ARG ILE THR ARG ILE PRO HIS LEU ILE ARG GLN ARG GLN PRO MET TYR ARG ASP GLY
 ARG GLY LEU ARG VAL TYR ARG ILE *** TYR GLY ARG ASP ASN GLN CYS THR GLY MET GLY
  ALA ASP TYR ALA TYR THR ALA SER ASP THR ALA GLU THR THR ASN VAL GLN GLY TRP ALA
CCGCGGATTACGCGTATACCGCATCTGATACGGCAGAGACAACCAATGTACAGGGATGGG
         1270      1280      1290      1300      1310      1320

LEU CYS LEU PRO ASN TYR THR ARG GLU GLY *** LYS *** HIS LEU *** CYS     LEU SER
 CYS VAL TYR GLN ILE THR HIS GLY LYS ALA GLU ASN ASP THR PHE ASP VAL     *** ALA
  VAL PHE THR LYS LEU HIS THR GLY ARG LEU LYS MET THR PRO LEU MET SER     LYS ARG
CTGTGTTTACCAAATTACACACGGGAAGGCTGAAAATGACACCTTTGATGTC--CTAAGC
         1330      1340      1350      1360      1370      1380

ALA SER GLN LEU THR PRO GLY HIS LYS PRO LEU LEU LEU GLY SER PRO GLN THR LEU SER
 PRO PRO ASN *** PRO PRO ASP THR ASN HIS TYR TYR TRP GLY VAL ARG ARG PRO CYS HIS
  LEU PRO ILE ASP PRO ARG THR GLN | THR THR THR THR GLY GLU SER ALA ASP PRO VAL THR
GCCTCCCAATTGACCCCCGGACACAA|ACCACTACTACTGGGGAGTCCGCAGACCCTGTCA
         1390      1400      1410      1420      1430      1440
                        VP3|VP1

PRO PRO PRO TRP ARG THR THR ALA VAL ILE HIS LYS SER ARG ASP VAL THR THR ARG THR
 HIS HIS ARG GLY GLU LEU ARG ARG *** TYR THR SER PRO GLU THR SER PRO HIS GLY ARG
  THR THR VAL GLU ASN TYR GLY GLY ASP THR GLN VAL GLN ARG ARG HIS HIS THR ASP VAL
CCACCACCGTGGAGAACTACGGCGGTGATACACAAGTCCAGAGACGTCACCACACGGACG
         1450      1460      1470      1480      1490      1500

SER ALA SER LEU TRP THR ASP LEU *** ARG *** THR ALA *** ALA PRO HIS MET SER LEU
 ARG LEU HIS TYR GLY PRO ILE CYS GLU ASP LYS GLN PRO GLU PRO HIS THR CYS HIS ***
  GLY PHE ILE MET ASP ARG PHE VAL LYS ILE ASN SER LEU SER PRO THR HIS VAL ILE ASP
TCGGCTTCATTATGGACCGATTTGTGAAGATAAACAGCCTGAGCCCCACACATGTCATTG
         1510      1520      1530      1540      1550      1560

THR SER CYS LYS PRO THR ASN THR GLY SER TRP VAL ARG TYR CYS VAL GLN PRO ARG THR
 PRO HIS ALA ASN PRO GLN THR ARG ASP ARG GLY CYS VAL THR ALA CYS SER HIS VAL LEU
  LEU MET GLN THR HIS LYS HIS GLY ILE VAL GLY ALA LEU LEU ARG ALA ALA THR TYR TYR
ACCTCATGCAAACCCACAAACACGGGATCGTGGGTGCGTTACTGCGTGCAGCCACGTACT
         1570      1580      1590      1600      1610      1620
```

12d

```
THR   SER   PRO   THR   TRP   ARG   LEU   LEU   CYS   GLY   THR   MET   VAL            ***   LEU   GLY   ALA
  LEU   LEU   ARG   LEU   GLY   ASP   CYS   CYS   ALA   ALA   ARG   TRP   ***            ASP   TRP   VAL   PRO
    PHE   SER   ASP   LEU   GLU   ILE   VAL   VAL   ARG   HIS   ASP   GLY            LEU   THR   GLY   CYS   PRO
A C T T C T C C G A C T T G G A G A T T G T T G T G C G G C A C G A T G G T A A - C T G A C T G G G T G C C C - - - - -
            1630                1640                1650                1660                1670                1680

- - - - - - - - - - - - - - - - - - - - - - - - - - - - - - - - - - - - - - - - - - - - - - - - - - - - - - - - - - - -
            1690                1700                1710                1720                1730                1740

                                      ARG   HIS   THR   ALA   CYS   TRP   GLN   LEU   CYS   THR   THR   GLY
                                        ALA   THR   PRO   ARG   VAL   GLY   ASN   CYS   VAL   ARG   ARG   ASP
                                          PRO   HIS   ARG   VAL   LEU   ALA   THR   VAL   TYR   ASP   GLY   THR
- - - - - - - - - - - - - - - - - - - - - C G C C A C A C C G C G T G T T G G C A A C T G T G T A C G A C G G G A
            1750                1760                1770                1780                1790                1800

GLN   THR   SER   THR   PRO   GLN   ARG
  LYS   GLN   VAL   LEU   ARG   SER   ASP
    ASN   LYS   TYR   SER   ALA   ALA
C A A A C A A G T A C T C C G C A G C G A T - - - - - - - - - - - - - - - - - - - - - - - - - - - - - - - - - - - - - -
            1810                1820                1830                1840                1850                1860

- - - - - - - - - - - - - - - - - - - - - - - - - - - - - - - - - - - - - - - - - - - - - - - - - - - - - - - - - - - -
            1870                1880                1890                1900                1910                1920

- - - - - - - - - - - - - - - - - - - - - - - - - - - - - - - - - - - - - - - - - - - - - - - - - - - - - - - - - - - -
            1930                1940                1950                1960                1970                1980

- - - - - - - - - - - - - - - - - - - - - - - - - - - - - - - - - - - - - - - - - - - - - - | - - - - - - - - - - - - -
            1990                2000                2010                2020                2030                2040
```

CORRECT READING FRAME UNKNOWN

POSSIBLE C-TERMINAL END OF VP1

FIG.13

13a

```
 ILE GLY THR ALA SER ARG PRO SER GLU VAL CYS MET VAL ASP
TATCGGTACCGCCTCGCGACCCAGCGAGGTGTGTATGGTAGA
20 30 40 50 60

GLY THR ASP MET CYS LEU ALA ASP PHE HIS ALA GLY ILE PHE MET LYS GLY GLN GLU HIS
CGGCACGGACATGTGTCTTGCTGATTTCCACGCAGGCATTTTCATGAAAGGACAGGAACA
70 80 90 100 110 120

ALA VAL PHE ALA CYS VAL THR SER ASP GLY TRP TYR ALA ILE ASP ASP GLU ASP PHE TYR
CGCAGTGTTCGCGTGTGTCACCTCAGACGGGTGGTACGCGATTGACGACGAGGACTTTTA
130 140 150 160 170 180

PRO TRP THR PRO ASP PRO SER ASP VAL LEU VAL PHE VAL PRO TYR ASP GLN GLU PRO LEU
CCCGTGGACGCCTGACCCATCGGACGTCTTGGTATTTGTCCCGTACGATCAAGAACCACT
190 200 210 220 230 240
p20
ASN GLY ASP TRP LYS THR GLN VAL GLN LYS LYS LEU LYS GLY ALA GLY GLN SER SER PRO
CAATGGGGACTGGAAAACACAGGTTCAGAAGAAGCTCAAGGGTGCTGGGCAGTCCAGCCC
250 260 270 280 290 300
POSITION OF N-TERMINAL OF VP1
ALA THR GLY SER GLN ASN GLN SER GLY ASN THR GLY SER ILE ILE ASN ASN TYR TYR MET
AGCAACCGGCTCGCAGAACCAGTCTGGCAACACTGGCAGCATAATTAACAACTACTACAT
310 320 330 340 350 360

GLN GLN TYR GLN ASN SER MET SER THR GLN LEU GLY ASP ASN THR ILE SER GLY GLY SER
GCAGCAATACCAGAACTCTATGAGCACACAGCTTGGTGACAATACCATCAGTGGAGGCTC
370 380 390 400 410 420

ASN GLU GLY SER THR ASP THR THR SER THR HIS THR THR ASN THR GLN ASN ASN ASP TRP
CAACGAGGGCTCCACGGACACAACTTCAACACACACAACCAACACCCAAAACAACGACTG
430 440 450 460 470 480
                                                                 VP4 | VP2
PHE SER LYS LEU ALA SER SER ALA PHE THR GLY LEU PHE GLY ALA LEU LEU ALA | ASP LYS
GTTTTCAAAACTTGCCAGTTCGGCTTTTACCGGTCTGTTCGGTGCACTTCTCGCC|GACAA
490 500 510 520 530 540

LYS THR GLU GLU THR THR LEU LEU GLU ASP ARG ILE LEU THR THR ARG ASN GLY HIS THR
GAAGACGGAAGAGACTACGCTTCTGGAAGACCGCATCCTCACTACCCGCAACGGGCACAC
550 560 570 580 590 600
```

CONT'D 13b ETC.

13b

```
 THR SER THR THR GLN SER SER VAL GLY VAL THR TYR GLY TYR SER THR GLU GLU ASP HIS
CACTTCGACCACCCAGTCGAGTGTGGGAGTCACGTATGGGTACTCCACTGAGGAAGATCA
         610       620       630       640       650       660

 VAL ALA GLY PRO ASN THR SER GLY LEU GLU THR ARG VAL VAL GLN ALA GLU ARG PHE PHE
CGTTGCTGGGCCCAACACATCGGGCTTAGAGACGCGGGTGGTGCAGGCAGAGAGATTTTT
         670       680       690       700       710       720

 LYS LYS PHE LEU PHE ASP TRP THR THR ASP LYS PRO PHE GLY TYR LEU THR LYS LEU GLU
CAAGAAGTTTCTGTTTGACTGGACAACGGACAAACCTTTTGGATACTTGACAAAACTGGA
         730       740       750       760       770       780

 LEU PRO THR ASP HIS HIS GLY VAL PHE GLY HIS LEU VAL ASP SER TYR ALA TYR MET ARG
GCTTCCCACCGATCACCACGGTGTCTTCGGGCACTTGGTGGACTCATATGCATATATGAG
         790       800       810       820       830       840

 ASN GLY TRP ASP VAL GLU VAL CYS ALA VAL GLY ASN GLN PHE ASN GLY GLY CYS LEU LEU
GAACGGCTGGGATGTTGAGGTATGTGCCGTCGGCAACCAGTTCAACGGCGGGTGCCTTCT
         850       860       870       880       890       900

 VAL ALA MET VAL PRO GLU TRP LYS ALA PHE ASP THR ARG GLU LYS TYR GLN LEU THR LEU
GGTGGCCATGGTGCCAGAGTGGAAGGCATTTGACACACGTGAAAAATACCAGCTTACCCT
         910       920       930       940       950       960

 PHE PRO HIS GLN PHE ILE SER PRO ARG THR ASN MET THR ALA HIS ILE THR VAL PRO TYR
TTTCCCACACCAGTTTATTAGCCCCAGAACTAACATGACTGCCCACATCACGGTACCGTA
         970       980       990      1000      1010      1020

 LEU GLY VAL ASN ARG TYR ASP GLN TYR LYS LYS HIS LYS PRO TRP THR LEU VAL VAL MET
TCTTGGTGTGAACAGGTACGATCAGTACAAGAAACACAAACCTTGGACACTGGTTGTCAT
        1030      1040      1050      1060      1070      1080

 VAL LEU SER PRO LEU THR VAL SER ASN THR ALA ALA PRO GLN ILE LYS VAL TYR ALA ASN
GGTACTATCACCCCTCACGGTCAGCAACACTGCCGCCCCACAAATCAAGGTCTACGCCAA
        1090      1100      1110      1120      1130      1140

                                                             VP2|VP3
 ILE ALA PRO THR TYR VAL HIS VAL ALA GLY GLU LEU PRO SER LYS GLU|GLY ILE PHE PRO
CATTGCCCCAACCTACGTTCACGTGGCTGGAGAGCTTCCCTCGAAAGAG|GGAATTTTCCC
        1150      1160      1170      1180      1190       1200

 VAL ALA CYS ALA ASP GLY TYR GLY GLY LEU VAL THR THR ASP PRO LYS THR ALA ASP PRO
AGTTGCATGCGCAGACGGTTACGGAGGACTGGTGACAACAGACCCGAAAACAGCTGACCC
        1210      1220      1230      1240      1250      1260

 VAL TYR GLY LYS VAL TYR ASN PRO PRO LYS THR ASN TYR PRO GLY ARG PHE THR ASN LEU
TGTTTACGGTAAGGTGTATAACCCGCCCAAGACCAACTACCCCGGGCGCTTTACAAACCT
        1270      1280      1290      1300      1310      1320

 LEU ASP VAL ALA GLU ALA CYS PRO THR PHE LEU ARG PHE ASP ASP GLY LYS PRO TYR VAL
ATTGGACGTGGCCGAAGCATGTCCCACCTTTCTTCGTTTCGACGATGGGAAACCGTACGT
        1330      1340      1350      1360      1370      1380
```

13c

```
 VAL THR ARG ALA ASP ASP THR ARG LEU LEU ALA LYS PHE ASP VAL SER LEU ALA ALA LYS
CGTTACGCGGGCAGACGACACCCGTCTTTTGGCCAAGTTTGATGTCTCCCTTGCCGCAAA
        1390      1400      1410      1420      1430      1440

 HIS MET SER ASN THR TYR LEU SER GLY ILE ALA GLN TYR TYR THR GLN TYR SER GLY THR
ACACATGTCCAACACATACCTATCAGGGATTGCACAGTACTACACACAGTACTCTGGTAC
        1450      1460      1470      1480      1490      1500

 ILE ASN LEU HIS PHE MET PHE THR GLY SER THR ASP SER LYS ALA ARG TYR MET VAL ALA
TATCAACCTGCACTTCATGTTCACAGGCTCCACTGACTCAAAAGCCCGCTACATGGTGGC
        1510      1520      1530      1540      1550      1560

 TYR ILE PRO LEU GLY VAL GLU THR PRO PRO ASP THR PRO GLU GLU ALA ALA HIS CYS ILE
TTACATCCCGCTCGGGGTGGAGACGCCGCCGGACACACCTGAAGAAGCTGCTCACTGCAT
        1570      1580      1590      1600      1610      1620

 HIS ALA GLU TRP ASP THR GLY LEU ASN SER LYS PHE THR PHE SER ILE PRO TYR VAL SER
TCACGCTGAGTGGGACACAGGACTGAACTCCAAATTCACCTTTTCAATCCCTTACGTGTC
        1630      1640      1650      1660      1670      1680

 ALA ALA ASP TYR ALA TYR THR ALA SER ASP THR ALA GLU THR THR ASN VAL GLN GLY TRP
TGCCGCGGATTACGCGTATACCGCATCTGATACGGCAGAGACAACCAATGTACAGGGATG
        1690      1700      1710      1720      1730      1740

 VAL CYS VAL TYR GLN ILE THR HIS GLY LYS ALA GLU ASN ASP THR LEU LEU VAL SER ALA
GGTCTGTGTTTACCAAATTACACACGGGAAGGCTGAAAATGACACCTTGTTAGTGTCGGC
        1750      1760      1770      1780      1790      1800
                                                             VP3|VP1
 SER ALA GLY LYS ASP PHE GLU LEU ARG LEU PRO ILE ASP PRO ARG THR GLN|THR THR THR
TAGCGCCGGCAAAGACTTTGAGTTGCGCCTCCCAATTGACCCCCGGACACAA|ACCACTAC
        1810      1820      1830      1840      1850      1860

 THR GLY GLU SER ALA ASP PRO VAL THR THR THR VAL GLU ASN TYR GLY GLY ASP THR GLN
TACTGGGGAGTCCGCAGACCCTGTCACCACCACCGTGGAGAACTACGGCGGTGATACACA
        1870      1880      1890      1900      1910      1920

 VAL GLN ARG ARG HIS HIS THR ASP VAL GLY PHE ILE MET ASP ARG PHE VAL LYS ILE ASN
AGTCCAGAGACGTCACCACACGGACGTCGGCTTCATTATGGACCGATTTGTGAAGATAAA
        1930      1940      1950      1960      1970      1980

 SER LEU SER PRO THR HIS VAL ILE ASP LEU MET GLN THR HIS LYS HIS GLY ILE VAL GLY
CAGCCTGAGCCCCACACATGTCATTGACCTCATGCAAACCCACAAACACGGGATCGTGGG
        1990      2000      2010      2020      2030      2040

 ALA LEU LEU ARG ALA ALA THR TYR TYR PHE SER ASP LEU GLU ILE VAL VAL ARG HIS ASP
TGCGTTACTGCGTGCAGCCACGTACTACTTCTCCGACTTGGAGATTGTTGTGCGGCACGA
        2050      2060      2070      2080      2090      2100

 GLY ASN LEU THR TRP VAL PRO ASN GLY ALA PRO GLU ALA ALA LEU SER ASN THR SER ASN
CGGTAATCTGACCTGGGTGCCCAACGGTGCCCCCGAGGCAGCCCTGTCAAACACCAGCAA
        2110      2120      2130      2140      2150      2160

 PRO THR ALA TYR ASN LYS ALA PRO PHE THR ARG LEU ALA LEU PRO TYR THR ALA PRO HIS
```

13d

```
CCCCACTGCCTACAACAAGGCACCGTTCACGAGACTTGCTCTCCCTTACACTGCGCCACA
         2170      2180      2190      2200      2210      2220

ARG VAL LEU ALA THR VAL TYR ASP GLY THR ASN LYS TYR SER ALA SER ASP SER ARG SER
CCGCGTGTTGGCAACTGTGTACGACGGGACAAACAAGTACTCCGCAAGCGATTCGAGATC
         2230      2240      2250      2260      2270      2280

GLY ASP LEU GLY SER ILE ALA ALA ARG VAL ALA THR GLN LEU PRO ALA SER PHE ASN TYR
AGGCGATCTGGGGTCCATCGCGGCGCGAGTCGCGACACAACTTCCTGCTTCCTTTAACTA
         2290      2300      2310      2320      2330      2340

GLY ALA ILE GLN ALA GLN ALA ILE HIS GLU LEU LEU VAL ARG MET LYS ARG ALA GLU LEU
CGGTGCAATCCAAGCACAGGCCATCCACGAGCTTCTCGTGCGCATGAAACGGGCCGAGCT
         2350      2360      2370      2380      2390      2400

TYR CYS PRO LYS PRO LEU LEU ALA ILE LYS VAL THR SER GLN ASP ARG TYR LYS GLN LYS
CTACTGTCCCAAGCCACTTCTAGCAATAAAGGTGACTTCGCAAGACAGGTACAAGCAAAA
         2410      2420      2430      2440      2450      2460

                           VP1 | p52
ILE ILE ALA PRO ALA LYS GLN LEU LEU | ASN PHE ASP LEU LEU LYS LEU ALA GLY ASP VAL
GATTATTGCGCCCGCAAAACAGCTGTTG|AACTTTGACCTACTTAAGTTGGCGGGTGACGT
         2470      2480      2490      2500      2510      2520

GLU SER ASN LEU GLY PRO PHE PHE PHE ALA ASP VAL ARG SER ASN PHE SER LYS LEU VAL
TGAGTCCAACCTTGGGCCCTTCTTCTTCGCTGACGTTAGGTCAAACTTTTCGAAGCTGGT
         2530      2540      2550      2560      2570      2580

ASP THR ILE ASN GLN MET GLN GLU ASP MET SER THR LYS HIS GLY PRO ASP PHE ASN ARG
AGACACCATCAATCAGATGCAGGAGGACATGTCCACAAAACACGGACCCGACTTTAACCG
         2590.     2600      2610      2620      2630      2640

LEU VAL SER ALA PHE GLU GLU LEU ALA THR GLY VAL LYS ALA ILE ARG THR GLY LEU ASP
GTTGGTGTCCGCTTTTGAGGAATTGGCCACTGGGGTTAAAGCTATCAGAACCGGTCTCGA
         2650      2660      2670      2680      2690      2700

GLU ALA LYS PRO TRP TYR LYS LEU ILE LYS LEU LEU SER ARG LEU SER CYS MET ALA ALA
TGAGGCCAAACCCTGGTACAAGCTCATCAAGCTCCTAAGCCGTCTGTCGTGCATGGCCGC
         2710      2720      2730      2740      2750      2760

VAL ALA ALA ARG SER LYS ASP PRO VAL LEU VAL ALA ILE MET LEU ALA ASP THR GLY LEU
TGTGGCAGCACGGTCCAAGGACCCAGTCCTTGTGGCCATCATGCTGGCCGACACCGGTCT
         2770      2780      2790      2800      2810      2820
```

FIG.14

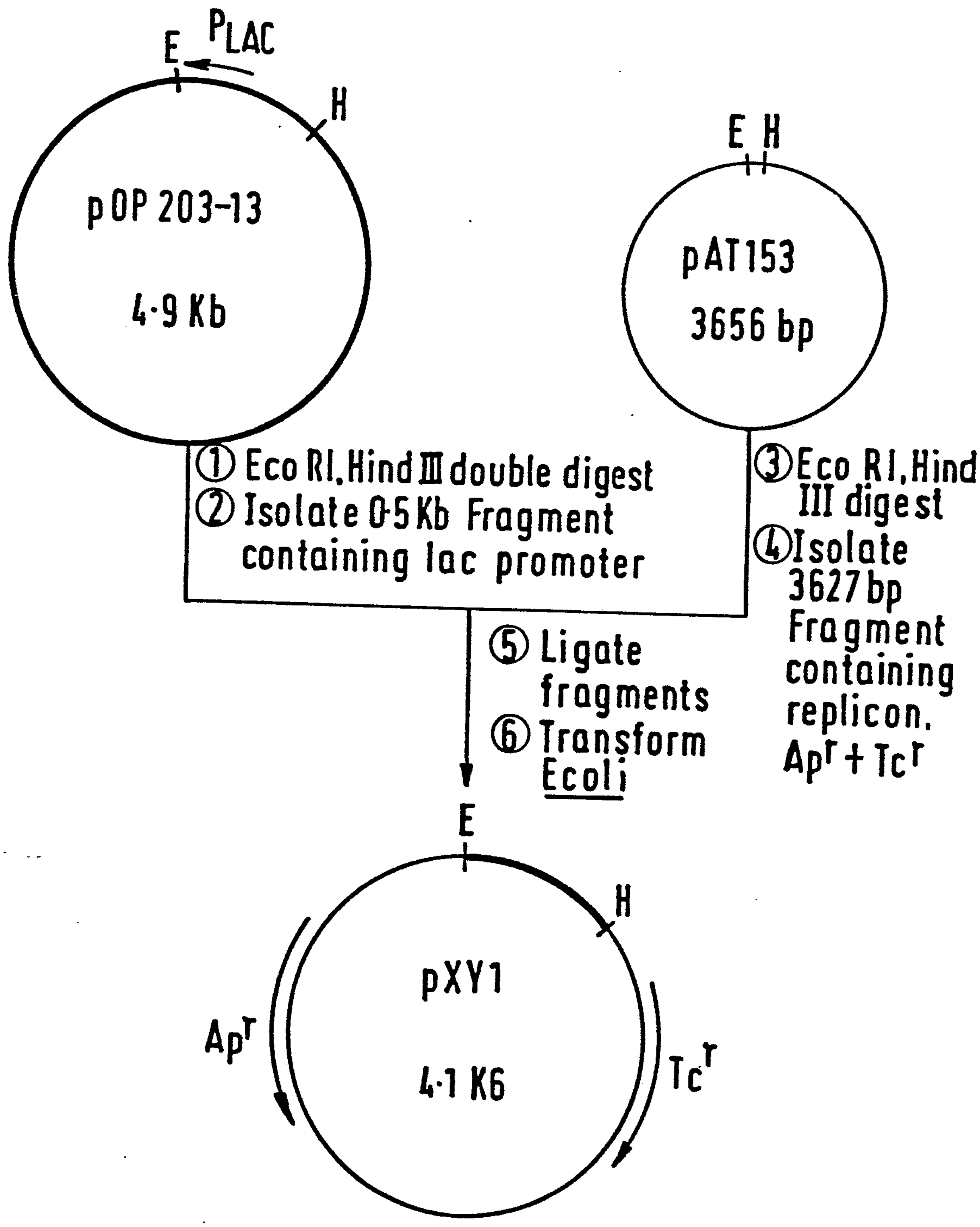
E
PLAC
H
pOP 203-13
4·9 Kb
E H
pAT153
3656 bp
① Eco RI, Hind III double digest
② Isolate 0·5 Kb Fragment containing lac promoter
③ Eco RI, Hind III digest
④ Isolate 3627 bp Fragment containing replicon. Apr + Tcr
⑤ Ligate fragments
⑥ Transform Ecoli
E
H
pXY1
4·1 K6
Apr
Tcr

FIG.15

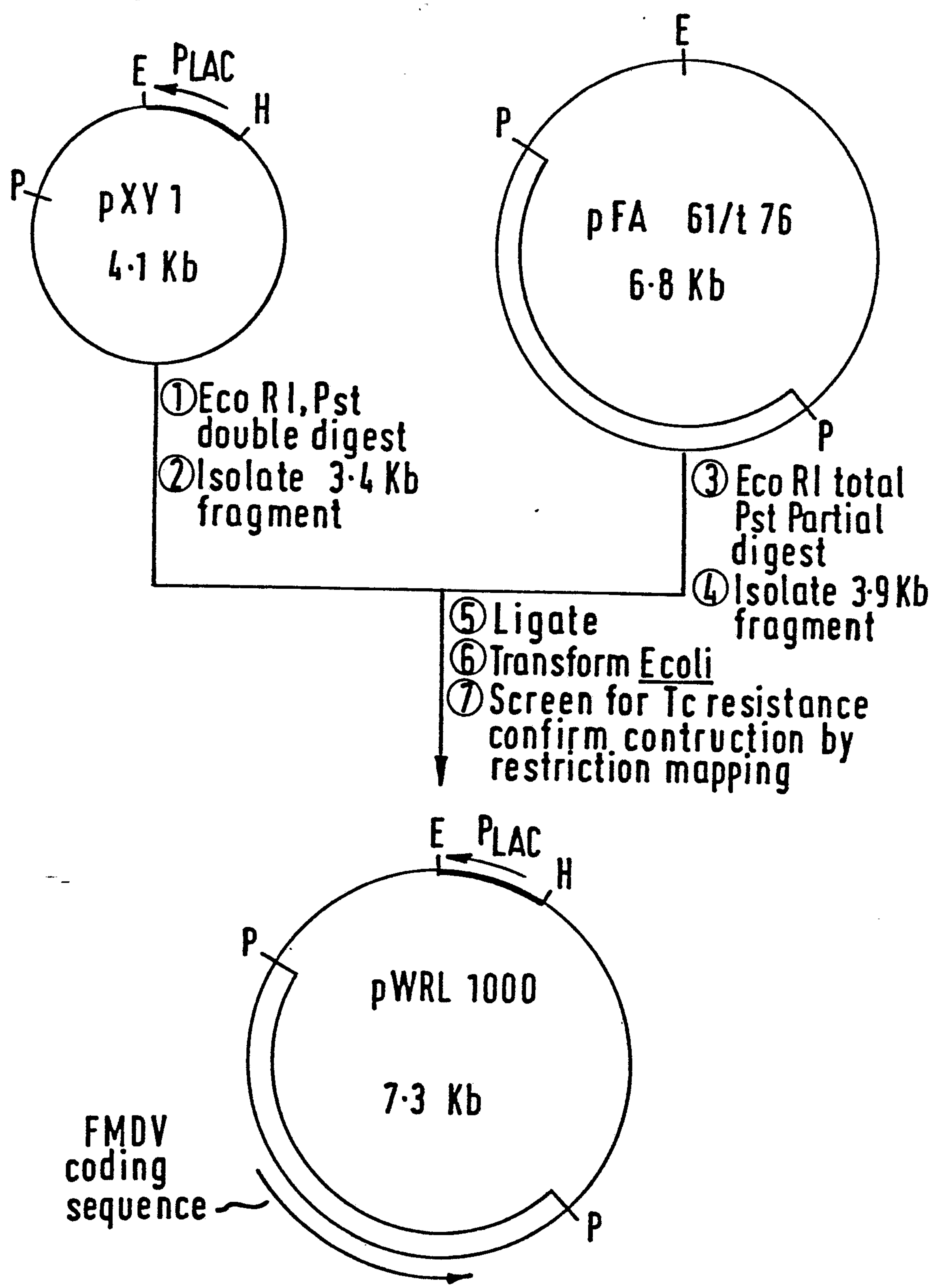
E
PLAC
H
P
pXY 1
4·1 Kb
E
P
pFA 61/t 76
6·8 Kb
P
①Eco R I, Pst
double digest
②Isolate 3·4 Kb
fragment
③Eco RI total
Pst Partial
digest
④Isolate 3·9 Kb
fragment
⑤Ligate
⑥Transform Ecoli
⑦Screen for Tc resistance
confirm contruction by
restriction mapping
E
PLAC
H
P
pWRL 1000
7·3 Kb
FMDV
coding
sequence
P

FIG.16

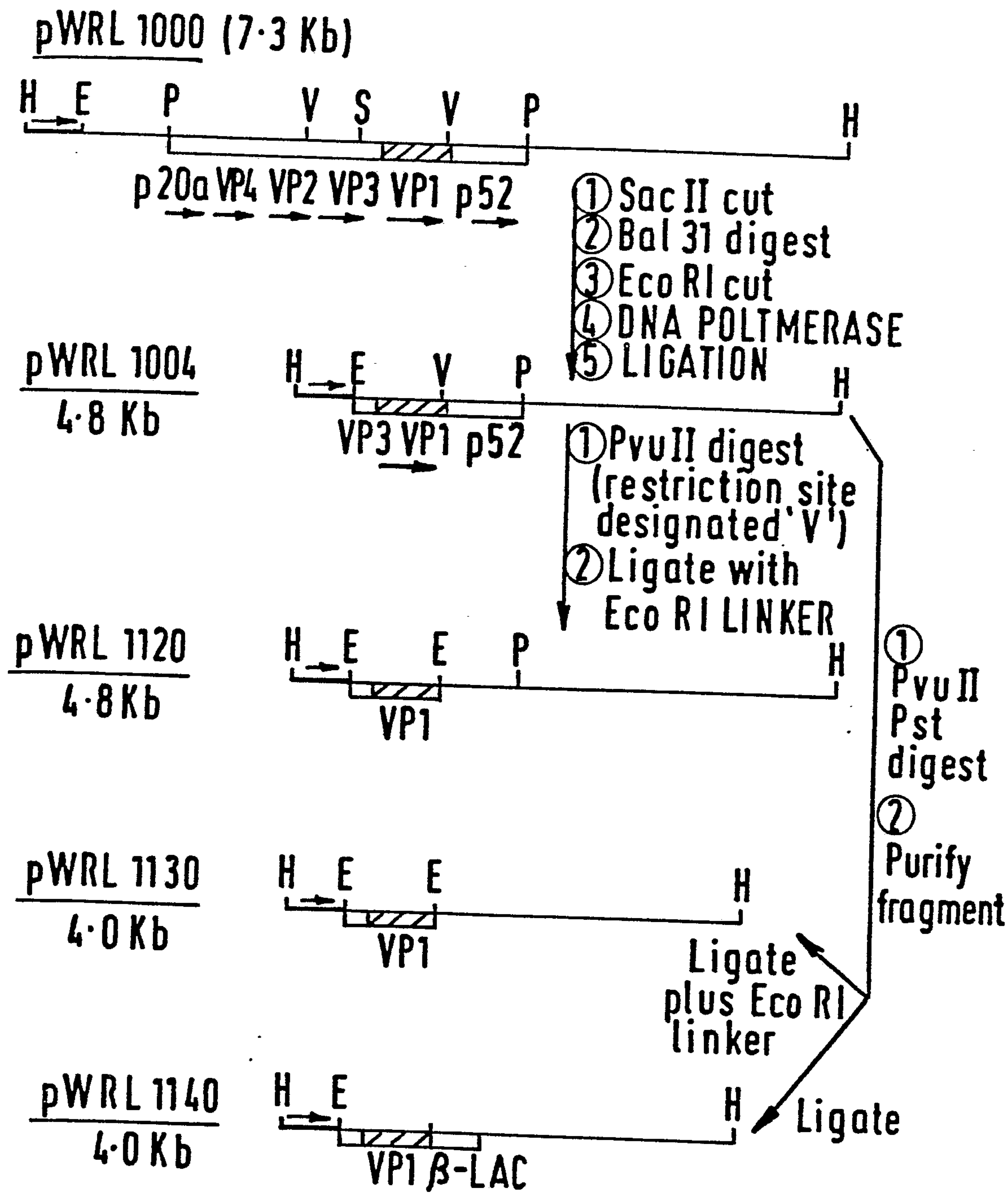
pWRL 1000 (7·3 Kb)
H
E
P
V
S
V
P
H
p20a VP4 VP2 VP3 VP1 p52
① Sac II cut
② Bal 31 digest
③ Eco RI cut
④ DNA POLTMERASE
⑤ LIGATION
pWRL 1004
4·8 Kb
VP3 VP1 p52
① PvuII digest (restriction site designated 'V')
② Ligate with Eco RI LINKER
pWRL 1120
4·8 Kb
VP1
① PvuII Pst digest
② Purify fragment
pWRL 1130
4·0 Kb
VP1
Ligate plus Eco RI linker
pWRL 1140
4·0 Kb
VP1 β-LAC
Ligate

FIG.17

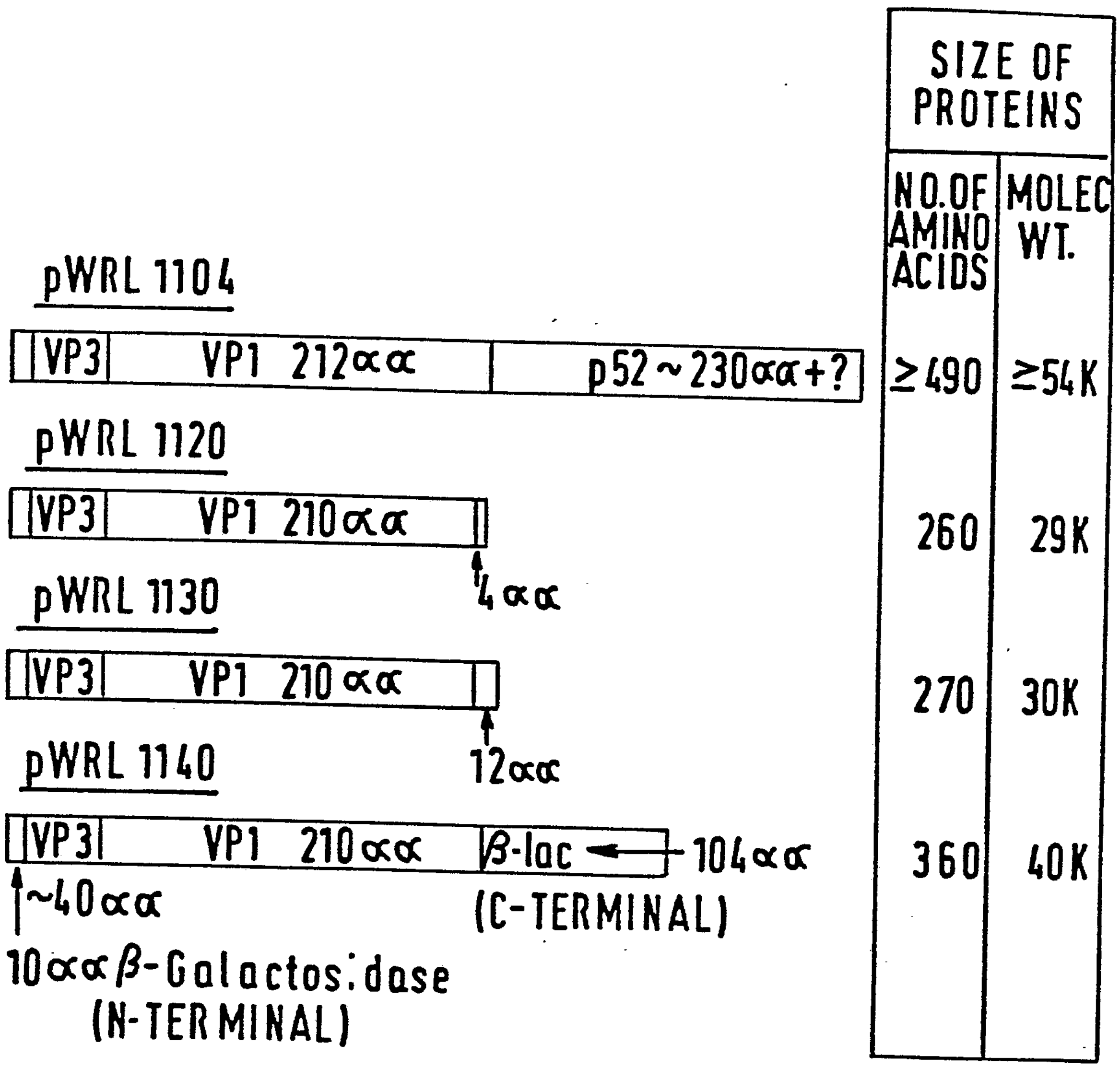
SIZE OF PROTEINS
NO.OF AMINO ACIDS
MOLEC WT.
pWRL 1104
VP3
VP1 212αα
p52 ~ 230αα+?
≥490
≥54K
pWRL 1120
VP3
VP1 210αα
4αα
260
29K
pWRL 1130
VP3
VP1 210 αα
12αα
270
30K
pWRL 1140
VP3
VP1 210αα
β-lac
104αα
(C-TERMINAL)
~40αα
10αα β-Galactosidase
(N-TERMINAL)
360
40K

FIG.18

① pWRL 1004

After translating the FMDV p52 sequences the ribosome will encounter a poly C (proline) sequence and terminate after 11, 32 or 106 amino acids depending on the reading frame.

② pWRL 1120 EcoRI LINKER

| | | | | | | |
|---|---|---|---|---|---|---|
| --- | CAG | GGA | ATT | CCC | TGT | TGA |
| | Gln | Gly | Ile | Pro | Cys | STOP |

VP1 αα210 ↑ (OUT-OF-FRAME FMDV SEQUENCE)

③ pWRL 1130 (OUT-OF-FRAME IN β-LACTAMASE)

| | | | | | | | |
|---|---|---|---|---|---|---|---|
| --- | CAG | GGA | ATT | CCG | CAA | TGG | Cont. |
| | Gln | Gly | Ile | Pro | Gln | Trp | — |

VP1 αα210

| | | | | | | | |
|---|---|---|---|---|---|---|---|
| -CAA | CAA | CGT | TGC | GCA | AAC | TAT | TAA |
| -Gln | Gln | Arg | Cys | Ala | Asn | Tyr | STOP |

④ pWRL 1140 (IN-FRAME WITH β-LACTAMASE)

| | | | | | | | | | |
|---|---|---|---|---|---|---|---|---|---|
| GCA | AAA | CAG | GCA | ATG | GCA | --- | CAT | TGG | TAA |
| Ala | Lys | Gln | Ala | Met | Ala | --- | His | Trp | STOP |

VP1 αα 210 ↑ — β-LACTAMASE αα183 ↑ — β-LACTAMASE αα286 ↑

# FIG.19

```
GACTCGCCGTTCCACTCTGGACCAGACGAGTACCGGCGCTCTTTGAGCCC
                                           ‾‾‾
                                            1
TTCTAGGGCTCTTTGAGATCCCAAGCTACAGATCACTTTACCTGCGTTGGG
   ‾‾‾       ‾‾‾
    2         3
TGAACGCCGTGTGCGGTGACGCATAATCCCCCAGAGATCAGAATTGGCACT
‾‾‾             ‾‾‾     ‾‾‾
 2               3       1
TCGGCTCTGGGGCGCGCGACGCCGTTAGGAGTGAAAAGCTCGA...(A)20
                         ‾‾‾    ‾‾‾
                          3      3
```

‾1‾ )
‾2‾ ) Stop codons in three reading phases
‾3‾ )

... Sequence unclear